# EUROPEAN PATENT APPLICATION

(11) **EP 4 843 482 A2**
(43) Date of publication of application: **25.11.2026**
(21) Application number: 26210236.1
(22) Date of filing: 14.09.2023
(51) Int. Cl.: A61B 5/00

(54) **SYSTEMS, DEVICES, AND METHODS FOR DUAL ANALYTE SENSOR**

(30) Priority: 15.09.2022 US 202263406989 P
(62) Document number(s) of the earlier application(s) in accordance with Art. 76 EPC: 23789403.5 / 4 586 907
(71) Applicant: Abbott Diabetes Care Inc., Alameda, CA 94502 (US)
(72) Inventor: BUDIMAN, Erwin S., Fremont, 94555 (US); CHO, Hyun, Berkeley, 94704 (US)
(74) Representative: J A Kemp LLP

(57) **Abstract**

Systems, devices, and methods for a dual analyte sensor using glucose history from a glucose sensor in combination with data from a ketone sensor to control operation of a user interface device or insulin pump are provided. In some embodiments, the systems, apparatus or methods may make use of combination of glucose history and a β-hydroxybutyrate physiological model to better predict diabetic ketoacidosis (DKA), in comparison to a prediction based on a simple high glucose threshold. In other embodiments, the systems, apparatus or method may include features for generating an estimate of the patient's medication state and/or knowledge of medication information, such as a patient with T1 diabetes mellitus (DM) using an SGLT-2 inhibitor.

## Description

### CROSS-REFERENCE TO RELATED APPLICATIONS

This application claims priority to U.S. Provisional Application No. 63/406,989, filed September 15, 2022, which is hereby expressly incorporated by reference in its entirety for all purposes.

### FIELD

The subject matter described herein relates generally to systems, devices, and methods for a dual analyte sensor. In particular, the embodiments described herein involve using data collected by a glucose sensor with data collected from a ketone sensor, for controlling a user interface device or dose administration device for improved control of a patient's glucose level.

### BACKGROUND

A vast and growing market exists for monitoring the health and condition of humans and other living animals. Information that describes the physical or physiological condition of humans can be used in countless ways to assist and improve quality of life and diagnose and treat undesirable human conditions.

A common device used to collect such information is a physiological sensor such as a biochemical analyte sensor, or a device capable of sensing a chemical analyte of a biological entity. Biochemical sensors come in many forms and can be used to sense analytes in fluids, tissues, or gases forming part of, or produced by, a biological entity, such as a human being. These analyte sensors can be used on or within the body itself, such as in the case of a transcutaneously implanted analyte sensor, or they can be used on biological substances that have already been removed from the body. Useful applications for such sensors include blood glucose sensing for purpose of health assessment, dose guidance, and related uses.

However, blood glucose monitoring alone is subject to certain limitations. For example, T1 diabetes patients using SGLT-2 inhibitors, also called gliflozins or flozins, are at risk of experiencing euglycemic diabetic ketoacidosis (DKA). DKA is an adverse condition concerning to diabetes patients, which can result in hospitalization or even death. It is associated with high ketone levels that are caused by long durations of high glucose levels. DKA can also be caused by insufficient insulin levels in the patient or high levels of insulin resistance, perhaps caused by illness - in this case, the glucose levels may be in the target range or below.

SGLT-2 is a diabetes medication that helps reduce glucose variability around mealtimes and is used for patients with T2 diabetes. It also can help patients with T1 diabetes in managing their glucose levels; however, there is a concern in using it for T1 patients because of the possibility of causing high ketone levels and DKA with normal levels of glucose, referred to herein as euglycemic DKA.

Treatment for euglycemic DKA is basically to administer insulin and to offset any unwanted glucose lowering impact by consuming carbohydrates. However, it can be confusing to the patients when they should do this and when they should seek emergency medical intervention. Specifically, it can be confusing to know what to do when the ketones are elevated enough to represent the euglycemic condition.

Discrete ketone test strips, along with continuous glucose monitoring (CGM), are available but may not be practical for continuous monitoring of ketones. Regardless of how the patient's ketone is measured, interpretation of the ketone level in conjunction with the patient's blood glucose levels and determination of appropriate action is too complex for most patients, requiring input from a health care provider (HCP). Accordingly, ketone sensing and use of ketone data by patients taking SGLT-2 inhibitors is relatively difficult and cumbersome, compared to continuous glucose sensing.

For these and other reasons, needs exist for improving ketone sensing, analysis and guidance for patients vulnerable to euglycemic DKA, for example, patients taking SGLT-2 inhibitors.

### SUMMARY

Example embodiments of systems, devices, and methods are described herein for a dual analyte sensor using glucose history from a glucose sensor in combination with data from a ketone sensor to control operation of a user interface device or insulin pump.

The present disclosure describes mobile app-based systems, apparatus and methods for detecting conditions where actions should be taken, providing guidance to the patient and providing a means to record important context concurrent with the condition that will be helpful for the HCP to know later when advising the patient how to avoid the adverse condition in the future. In an aspect, the systems, apparatus or methods may make use of combination of glucose history and a β-hydroxybutyrate physiological model to better predict diabetic ketoacidosis (DKA), in comparison to a prediction based on a simple high glucose threshold. In an alternative, or in addition, the systems, apparatus or method may include features for generating an estimate of the patient's medication state and/or knowledge of medication information, such as a patient with T1 diabetes mellitus (DM) using an SGLT-2 inhibitor.

In addition, or in an alternative, an improved system, method or apparatus may include improving the alert feature of an analyte monitoring sensor (e.g. glucose sensor) by using context from 1 or more additional analyte sensor (e.g. ketone sensor) and/or estimate of medication state and/or knowledge of medication information (e.g. person with T1DM using SGLT-2 inhibitor). Single analyte sensor systems may have various alerts. Examples of alerts include high threshold alert, and low projected threshold alert. If at least 1 more analyte information and/or medication information is known, the alert can be improved by adjusting the alert behavior and timing. This may include dual analyte systems (e.g. Glucose-Ketone) for which threshold alerts are based on each analyte's value, independent of other analyte values and/or medication based information. Examples of adjusting alert behavior include using a lower or higher threshold. An example of adjusting timing includes changing the alert enunciation time interval when the alert condition is still met. This improves the clinical relevance of the alert and may reduce alert fatigue by minimizing enunciation that may be less clinically relevant.

The systems, apparatus and methods disclosed herein incorporate ketone data together with blood glucose data to provide patient and HCP guidance that is more reliable than using high glucose threshold detection alone. On-demand systems or continuous glucose monitoring (CGM) systems may thus be provided with improved utility. For example, an on-demand test system including a built-in ketone-measurement-compatible strip port can provide an enhanced utility to the patient and assist the HCP in making more accurate recommendations. In general, the systems, apparatus and methods disclosed herein may better protect the patient from DKA risk. Algorithmic improvements in the systems, methods and apparatus may include utilization of rich glucose history from on-demand or CGM systems, opportunistic use of insulin history (e.g. from a built-in bolus calculator) and ongoing ketone measurement (e.g. from built-in ketone compatible strip port or in vivo ketone analyte sensor) to improve future DKA risk estimation, and improved DKA risk assessment. Improved risk assessment algorithms may include, for example, comparing estimated ketone time series to a ketone-specific threshold, instead of comparing point glucose levels to a conservative point glucose specific threshold as is conventionally done.

According to some embodiments, an analyte monitoring system is provided in which a sensor control device is configured to collect first time-correlated data indicative of a glucose level and second time-correlated data indicative of a ketone level. For example, the first data may be from the analyte sensor, which is a glucose sensor, and the second data may be from the analyte sensor, which is also a ketone sensor. In other examples, the second data may be received, such as from a ketone test strip measurement. In some embodiments, one or more of the first data and the second data is from the analyte sensor.

According to some embodiments, the sensor control device is operatively coupled to at least one first processing circuitry and at least one first non-transitory memory. For example, the first data and/or the second data may be stored in one or more memories (e.g. in a single or separate memories). In some embodiments, the reader device comprises at least one second processing circuitry and at least one second non-transitory memory. For example, the first data and/or the second data may be stored in one or more memories (e.g. in a single or separate memories).

According to some embodiments, at least one of the non-transitory memories includes instructions which, when executed, cause at least one of the processing circuitry in the sensor control device or the reader device to make a determination based on the first and second time-correlated data and output, by the reader device, an indication of the determination. The determination may be at least one of an alert threshold for one or both of the first and second time-correlated data, a message for output by the reader device, and/or a correction to an analyte state estimate. Determining an alert threshold may include, for example, setting or modifying a threshold value for blood glucose, for ketone bodies, or for another analyte, which when exceed causes the reader device or another system component to output an alarm. Determining a message may include, for example, selecting a predetermined message from a data table in response to a result of automatic analysis of the first and second time-correlated data. Determining a correction to an analyte state estimate may include, for example, calculating a correction factor for, or corrected value of, an initial estimate for blood glucose or other analyte made based on the first time-correlated data only.

Other systems, devices, methods, features and advantages of the subject matter described herein will be or will become apparent to one with skill in the art upon examination of the following figures and detailed description. It is intended that all such additional systems, methods, features and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is an illustrative view depicting an example embodiment of an in vivo analyte monitoring system.
FIG. 2 is a block diagram of an example embodiment of a reader device.
FIG. 3 is a block diagram of an example embodiment of a sensor control device.
FIGS. 4A, 4B and 4C are multi-plot graphs depicting example analyte concentrations measured over time.
FIG. 5 is a flow diagram depicting an example embodiment of a method for analyte monitoring.
FIGS. 6-10 are flow diagrams depicting alternative embodiments and aspects of the method diagrammed in FIG. 5.

### DETAILED DESCRIPTION

Before the present subject matter is described in detail, it is to be understood that this disclosure is not limited to the particular embodiments described, as such may vary. It is also to be understood that the terminology used herein is for the purpose of describing particular embodiments only and is not intended to be limiting. The scope of the present disclosure will be limited only by the appended claims.

The publications discussed herein are provided solely for their disclosure prior to the filing date of the present application. Nothing herein is to be construed as an admission that the present disclosure is not entitled to antedate such publications by virtue of prior disclosure. Furthermore, the dates of publication provided may be different from the actual publication dates, which may need to be independently confirmed.

Generally, embodiments of the present disclosure are used with systems, devices, and methods for detecting at least one analyte, such as glucose, in a bodily fluid (e.g., subcutaneously within the interstitial fluid ("ISF") or blood, within the dermal fluid of the dermal layer, or otherwise), in conjunction with a feature for ketone analyte sensing that is chronologically correlated to the analyte data from an in vivo glucose sensor. Embodiments may include in vivo analyte sensors structurally configured so that at least a portion of the sensor is, or can be, positioned in the body of a user to obtain information about at least one analyte of the body. However, the embodiments disclosed herein can be used with in vivo analyte monitoring systems that incorporate in vitro capability, as well as purely in vitro or ex vivo analyte monitoring systems, including those systems that are entirely non-invasive. If used with a single-analyte in vivo sensor, ketone test data may be added manually, for example by using a test strip. In an alternative, embodiments of the present disclosure may be used with dual-sensor systems for continuous or semi-continuous monitoring of different analytes, for example blood glucose and ketone bodies.

Furthermore, for each embodiment of a method disclosed herein, systems and devices capable of performing each of those embodiments are covered within the scope of the present disclosure. For example, embodiments of sensor control devices are disclosed and these devices can have one or more sensors, analyte monitoring circuitry (e.g., an analog circuit), non-transitory memories (e.g., for storing instructions), power sources, communication circuitry, transmitters, receivers, processing circuitry, and/or controllers (e.g., for executing instructions) that can perform any and all method steps or facilitate the execution of any and all method steps. These sensor control device embodiments can be used and can be capable of use to implement those steps performed by a sensor control device from one or more of the methods described herein.

Likewise, embodiments of reader devices are disclosed having one or more transmitters, receivers, non-transitory memories (e.g., for storing instructions), power sources, processing circuitry, and/or controllers (e.g., for executing instructions) that can perform any and all method steps or facilitate the execution of any and all method steps. These embodiments of the reader devices can be used to implement those steps performed by a reader device from one or more of the methods described herein.

Embodiments of trusted computer systems are also disclosed. These trusted computer systems can include one or more processing circuitry, controllers, transmitters, receivers, non-transitory memories, databases, servers, and/or networks, and can be discretely located or distributed across multiple geographic locales. These embodiments of the trusted computer systems can be used to implement those steps performed by a trusted computer system from one or more of the methods described herein.

Various embodiments of systems, devices and methods for improving the accuracy of an analyte sensor and for detecting sensor fault conditions are disclosed. According to some embodiments, these systems, devices, and methods can utilize a first data collected by a glucose sensor and a second data collected by a ketone sensing element.

Other features and advantages of the disclosed embodiments are further discussed below.

Before describing the embodiments in detail, however, it is first desirable to describe examples of devices that can be present within, for example, an in vivo analyte monitoring system, as well as examples of their operation, all of which can be used with the embodiments described herein.

### Example Embodiments of Analyte Monitoring Systems

There are various types of analyte monitoring systems. "Continuous Analyte Monitoring" systems (or "Continuous Glucose Monitoring" systems), for example, are in vivo systems that can transmit data from a sensor control device to a reader device repeatedly or continuously without prompting, e.g., automatically according to a schedule. "Flash Analyte Monitoring" systems (or "Flash Glucose Monitoring" systems or simply "Flash" systems), as another example, are in vivo systems that can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo monitoring systems can include a sensor that, while positioned in vivo, contacts the bodily fluid of the user and senses one or more analyte levels contained therein. The sensor can be part of a sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, for example. As used herein, these terms are not limited to devices with analyte sensors, and encompass devices that have sensors of other types, whether biometric or non-biometric. The term "on body" refers to any device that resides directly on the body or in close proximity to the body, such as a wearable device (e.g., glasses, watch, wristband or bracelet, neckband or necklace, etc.).

In vivo monitoring systems can also include one or more reader devices that receive sensed analyte data from the sensor control device. These reader devices can process and/or display the sensed analyte data, or sensor data, in any number of forms, to the user. These devices, and variations thereof, can be referred to as "handheld reader devices," "reader devices" (or simply, "readers"), "handheld electronics" (or handhelds), "portable data processing" devices or units, "data receivers," "receiver" devices or units (or simply receivers), "relay" devices or units, or "remote" devices or units, to name a few. Other devices such as personal computers have also been utilized with or incorporated into in vivo and in vitro monitoring systems.

In vivo analyte monitoring systems can be differentiated from "in vitro" systems that contact a biological sample outside of the body (or rather "ex vivo") and that typically include a meter device that has a port for receiving an analyte test strip carrying a bodily fluid of the user, which can be analyzed to determine the user's analyte level. As mentioned, the embodiments described herein can be used with in vivo systems, in vitro systems, and combinations thereof.

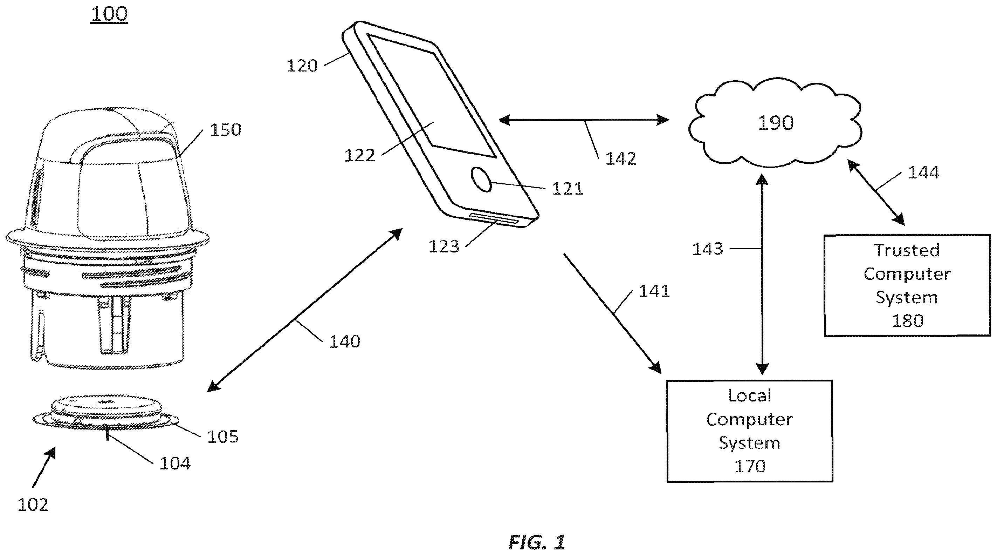

FIG. 1 is an illustrative view depicting an example embodiment of an in vivo analyte monitoring system 100 having a sensor control device 102 and a reader device 120 that communicate with each other over a local communication path (or link) 140, which can be wired or wireless, and uni-directional or bi-directional. In embodiments where path 140 is wireless, a near field communication (NFC) protocol, RFID protocol, Bluetooth or Bluetooth Low Energy protocol, Wi-Fi protocol, proprietary protocol, or the like can be used, including those communication protocols in existence as of the date of this filing or their later developed variants.

Reader device 120 is also capable of wired, wireless, or combined communication with a computer system 170 (e.g., a local or remote computer system) over communication path (or link) 141 and with a network 190, such as the internet or the cloud, over communication path (or link) 142. Communication with network 190 can involve communication with trusted computer system 180 within network 190, or though network 190 to computer system 170 via communication link (or path) 143. Communication paths 141, 142, and 143 can be wireless, wired, or both, can be uni-directional or bi-directional, and can be part of a telecommunications network, such as a Wi-Fi network, a local area network (LAN), a wide area network (WAN), the internet, or other data network. In some cases, communication paths 141 and 142 can be the same path. All communications over paths 140, 141, and 142 can be encrypted and sensor control device 102, reader device 120, computer system 170, and trusted computer system 180 can each be configured to encrypt and decrypt those communications sent and received.

Variants of devices 102 and 120, as well as other components of an in vivo-based analyte monitoring system that are suitable for use with the system, device, and method embodiments set forth herein, are described in U.S. Patent Publication No. 2011/0213225 (the '225 Publication), which is incorporated by reference herein in its entirety for all purposes.

Sensor control device 102 can include a housing 103 containing in vivo analyte monitoring circuitry and a power source. In this embodiment, the in vivo analyte monitoring circuitry is electrically coupled with one or more analyte sensors 104, 106 that extend through an adhesive patch 105 and projects away from housing 103. The sensors may include a blood glucose sensor 104 and a ketone sensor 106. In an alternative, a sensor capable for dual-analyte sensing may be configured to sense both glucose and ketone. For example, a dual-analyte sensor as disclosed in U.S. Patent Publication No. 2020/0237276 (the '276 Publication), which is incorporated by reference herein in its entirety for all purposes, may be used.

An adhesive patch 105 may include an adhesive layer (not shown) for attachment to a skin surface of the body of the user. Other forms of body attachment to the body may be used, in addition to or instead of adhesive.

A glucose sensor 104 and optionally, a ketone sensor 106, may be adapted to be at least partially inserted into the body of the user, where it can make fluid contact with that user's bodily fluid (e.g., subcutaneous (subdermal) fluid, dermal fluid, or blood) and be used, along with the in vivo analyte monitoring circuitry, to measure analyte-related data of the user. Sensors 104, 106 and any accompanying sensor control electronics can be applied to the body in any desired manner. For example, an insertion device 150 can be used to position all or a portion of analyte sensor 104 through an external surface of the user's skin and into contact with the user's bodily fluid. In doing so, the insertion device can also position sensor control device 102 with adhesive patch 105 onto the skin. In other embodiments, insertion device can position sensor 104 first, and then accompanying sensor control electronics can be coupled with sensor 104 afterwards, either manually or with the aid of a mechanical device. Examples of insertion devices are described in U.S. Publication Nos. 2008/0009692, 2011/0319729, 2015/0018639, 2015/0025345, and 2015/0173661, all which are incorporated by reference herein in their entireties and for all purposes.

After collecting raw data from the user's body, sensor control device 102 can apply analog signal conditioning to the data and convert the data into a digital form of the conditioned raw data. In some embodiments, sensor control device 102 can then algorithmically process the digital raw data into a form that is representative of the user's measured biometric (e.g., analyte level) and/or one or more analyte metrics based thereupon. For example, sensor control device 102 can include processing circuitry to algorithmically perform any of the method steps described herein. Sensor control device 102 can then encode and wirelessly communicate data indicative of a glucose level, a ketone level, indications of sensor fault and/or processed sensor data to reader device 120, which in turn can format or graphically process the received data for digital display to the user. In other embodiments, in addition to, or in lieu of, wirelessly communicating sensor data to another device (e.g., reader device 120), sensor control device 102 can graphically process the final form of the data such that it is ready for display, and display that data on a display of sensor control device 102. In some embodiments, the final form of the biometric data (prior to graphic processing) is used by the system (e.g., incorporated into a diabetes monitoring regime) without processing for display to the user.

In still other embodiments, the conditioned raw digital data can be encoded for transmission to another device, e.g., reader device 120, which then algorithmically processes that digital raw data into a form representative of the user's measured biometric (e.g., a form readily made suitable for display to the user) and/or one or more analyte metrics based thereupon. Reader device 120 can include processing circuitry to algorithmically perform any of the method steps described herein such as, for example, to correct a glucose level measurement, to detect a suspected glucose dropout, or to detect a suspected sensor fault condition, or other operation. This algorithmically processed data can then be formatted or graphically processed for digital display to the user.

In other embodiments, sensor control device 102 and reader device 120 transmit the digital raw data to another computer system for algorithmic processing and display.

Reader device 120 can include a display 122 to output information to the user and/or to accept an input from the user, and an optional input component 121 (or more), such as a button, actuator, touch sensitive switch, capacitive switch, pressure sensitive switch, jog wheel or the like, to input data, commands, or otherwise control the operation of reader device 120. In certain embodiments, display 122 and input component 121 may be integrated into a single component, for example, where the display can detect the presence and location of a physical contact touch upon the display, such as a touch screen user interface. In certain embodiments, input component 121 of reader device 120 may include a microphone and reader device 120 may include software configured to analyze audio input received from the microphone, such that functions and operation of the reader device 120 may be controlled by voice commands. In certain embodiments, an output component of reader device 120 includes a speaker (not shown) for outputting information as audible signals. Similar voice responsive components such as a speaker, microphone and software routines to generate, process and store voice driven signals may be included in sensor control device 102.

Reader device 120 can also include one or more data communication ports 123 for wired data communication with external devices such as computer system 170 or sensor control device 102. Example data communication ports include USB ports, mini USB ports, USB Type-C ports, USB micro-A and/or micro-B ports, RS-232 ports, Ethernet ports, Firewire ports, or other similar data communication ports configured to connect to the compatible data cables. Reader device 120 may also include an integrated or attachable in vitro glucose meter, including an in vitro test strip port (not shown) to receive an in vitro glucose test strip for performing in vitro blood glucose measurements.

Reader device 120 can display the measured biometric data wirelessly received from sensor control device 102 and can also be configured to output alarms, alert notifications, glucose values, etc., which may be visual, audible, tactile, or any combination thereof. Further details and other display embodiments can be found in, e.g., U.S. Publication No. 2011/0193704, which is incorporated herein by reference in its entirety for all purposes.

Reader device 120 can function as a data conduit to transfer the measured data and/or analyte metrics from sensor control device 102 to computer system 170 or trusted computer system 180. In certain embodiments, the data received from sensor control device 102 may be stored (permanently or temporarily) in one or more memories of reader device 120 prior to uploading to system 170, 180 or network 190.

Computer system 170 may be a personal computer, a server terminal, a laptop computer, a tablet, or other suitable data processing device. Computer system 170 can be (or include) software for data management and analysis and communication with the components in analyte monitoring system 100. Computer system 170 can be used by the user or a medical professional to display and/or analyze the biometric data measured by sensor control device 102. In some embodiments, sensor control device 102 can communicate the biometric data directly to computer system 170 without an intermediary such as reader device 120, or indirectly using an internet connection (also optionally without first sending to reader device 120). Operation and use of computer system 170 may be as further described in the '225 Publication incorporated herein, with additional method steps for handling ketone data in conjuction with blood glucose data. Analyte monitoring system 100 can also be configured to operate with a data processing module (not shown), also as described in the incorporated '225 Publication.

Trusted computer system 180 can be within the possession of the manufacturer or distributor of sensor control device 102, either physically or virtually through a secured connection, and can be used to perform authentication of sensor control device 102, for secure storage of the user's biometric data, and/or as a server that serves a data analytics program (e.g., accessible via a web browser) for performing analysis on the user's measured data.

### Example Embodiments of Reader Devices

Reader device 120 can be a mobile communication device such as a dedicated reader device (configured for communication with a sensor control device 102, and optionally a computer system 170, but without mobile telephony communication capability) or a mobile telephone including, but not limited to, a Wi-Fi or internet enabled smart phone, tablet, or personal digital assistant (PDA). Examples of smart phones can include those mobile phones based on a Windows^{®} operating system, Android^{™} operating system, iPhone^{®} operating system, Palm^{®} WebOS^{™}, Blackberry^{®} operating system, or Symbian^{®} operating system, with data network connectivity functionality for data communication over an internet connection and/or a local area network (LAN).

Reader device 120 can also be configured as a mobile smart wearable electronics assembly, such as an optical assembly that is worn over or adjacent to the user's eye (e.g., a smart glass or smart glasses, such as Google glasses, which is a mobile communication device). This optical assembly can have a transparent display that displays information about the user's analyte level (as described herein) to the user while at the same time allowing the user to see through the display such that the user's overall vision is minimally obstructed. The optical assembly may be capable of wireless communications similar to a smart phone. Other examples of wearable electronics include devices that are worn around or in the proximity of the user's wrist (e.g., a watch, etc.), neck (e.g., a necklace, etc.), head (e.g., a headband, hat, etc.), chest, or the like.

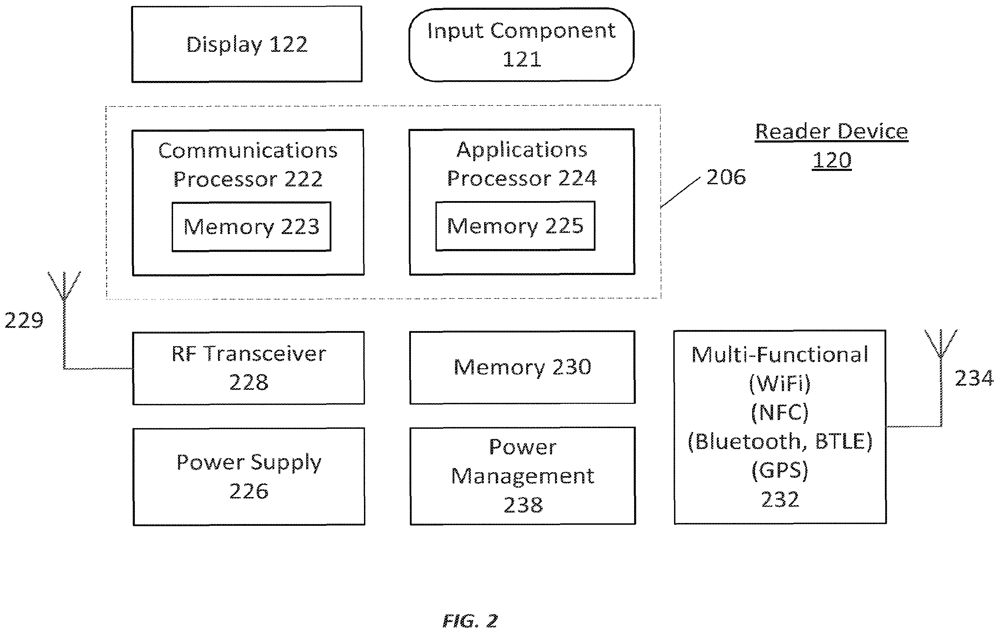

FIG. 2 is a block diagram of an example embodiment of a reader device 120 configured as a smart phone. Here, reader device 120 includes an input component 121, display 122, and processing circuitry 206, which can include one or more processors, microprocessors, controllers, and/or microcontrollers, each of which can be a discrete chip or distributed amongst (and a portion of) a number of different chips. Here, processing circuitry 206 includes a communications processor 222 having on-board memory 223 and an applications processor 224 having on-board memory 225. Reader device 120 further includes RF communication circuitry 228 coupled with an RF antenna 229, a memory 230, multi-functional circuitry 232 with one or more associated antennas 234, a power supply 226, power management circuitry 238, and a clock (not shown). One or more of the memories 223, 225 may hold program instructions, that when executed by one or more of the processors 222, 224, cause the reader device 120 to perform one or more operations of methods described herein. FIG. 2 is an abbreviated representation of the typical hardware and functionality that resides within a smart phone and those of ordinary skill in the art will readily recognize that other hardware and functionality (e.g., codecs, drivers, glue logic) can also be included.

Communications processor 222 can interface with RF communication circuitry 228 and perform analog-to-digital conversions, encoding and decoding, digital signal processing and other functions that facilitate the conversion of voice, video, and data signals into a format (e.g., in-phase and quadrature) suitable for provision to RF communication circuitry 228, which can then transmit the signals wirelessly. Communications processor 222 can also interface with RF communication circuitry 228 to perform the reverse functions necessary to receive a wireless transmission and convert it into digital data, voice, and video. RF communication circuitry 228 can include a transmitter and a receiver (e.g., integrated as a transceiver) and associated encoder logic.

Applications processor 224 can be adapted to execute the operating system and any software applications that reside on reader device 120, process video and graphics, and perform those other functions not related to the processing of communications transmitted and received over RF antenna 229. The smart phone operating system will operate in conjunction with a number of applications on reader device 120. Any number of applications (also known as "user interface applications") can be running on reader device 120 at any one time, and may include one or more applications that are related to a diabetes monitoring regime and methods described herein, in addition to the other commonly used applications that are unrelated to such a regime, e.g., email, calendar, weather, sports, games, etc. For example, the data indicative of a sensed analyte level and in vitro blood analyte measurements received by the reader device can be securely communicated to user interface applications residing in memory 230 of reader device 120. Such communications can be securely performed, for example, by mobile application containerization or wrapping technologies.

Memory 230 can be shared by one or more of the various functional units present within reader device 120, or can be distributed amongst two or more of them (e.g., as separate memories present within different chips). Memory 230 can also be a separate chip of its own. Memories 223, 225, and 230 are non-transitory, and can be volatile (e.g., RAM, etc.) and/or non-volatile memory (e.g., ROM, flash memory, F-RAM, etc.).

Multi-functional circuitry 232 can be implemented as one or more chips and/or components (e.g., transmitter, receiver, transceiver, and/or other communication circuitry) that perform other functions such as local wireless communications, e.g., with sensor control device 102 under the appropriate protocol (e.g., Wi-Fi, Bluetooth, Bluetooth Low Energy, Near Field Communication (NFC), Radio Frequency Identification (RFID), proprietary protocols, and others) and determining the geographic position of reader device 120 (e.g., global positioning system (GPS) hardware). One or more other antennas 234 are associated with the functional circuitry 232 as needed to operate with the various protocols and circuits.

Power supply 226 can include one or more batteries, which can be rechargeable or single-use disposable batteries. Power management circuitry 238 can regulate battery charging and power supply monitoring, boost power, perform DC conversions, and the like.

Reader device 120 can also include or be integrated with a drug (e.g., insulin, etc.) delivery device such that they, e.g., share a common housing. Examples of such drug delivery devices can include medication pumps having a cannula that remains in the body to allow infusion over a multi-hour or multi-day period (e.g., wearable pumps for the delivery of basal and bolus insulin). Reader device 120, when combined with a medication pump, can include a reservoir to store the drug, a pump connectable to transfer tubing, and an infusion cannula. The pump can force the drug from the reservoir, through the tubing and into the person with diabetes' body by way of the cannula inserted therein. Other examples of drug delivery devices that can be included with (or integrated with) reader device 120 include portable injection devices that pierce the skin only for each delivery and are subsequently removed (e.g., insulin pens). A reader device 120, when combined with a portable injection device, can include an injection needle, a cartridge for carrying the drug, an interface for controlling the amount of drug to be delivered, and an actuator to cause injection to occur. The device can be used repeatedly until the drug is exhausted, at which point the combined device can be discarded, or the cartridge can be replaced with a new one, at which point the combined device can be reused repeatedly. The needle can be replaced after each injection.

The combined device may function as part of a closed-loop system (e.g., an artificial pancreas system requiring no user intervention to operate) or semi-closed loop system (e.g., an insulin loop system requiring seldom user intervention to operate, such as to confirm changes in dose). For example, the person with diabetes' analyte level can be monitored in a repeated automatic fashion by sensor control device 102, which may then communicate that monitored analyte level to reader device 120, and the appropriate drug dosage to control the analyte level can be automatically determined and subsequently delivered to the person with diabetes' body. Software instructions for controlling the pump and the amount of insulin delivered may be stored in the memory of reader device 120 and executed by the reader device's processing circuitry. These instructions can also cause calculation of drug delivery amounts and durations (e.g., a bolus infusion and/or a basal infusion profile) based on the analyte level measurements obtained directly or indirectly from sensor control device 102. In some embodiments sensor control device 102 may determine the drug dosage and communicate that to reader device 120.

### Example Embodiments of Sensor Control Devices

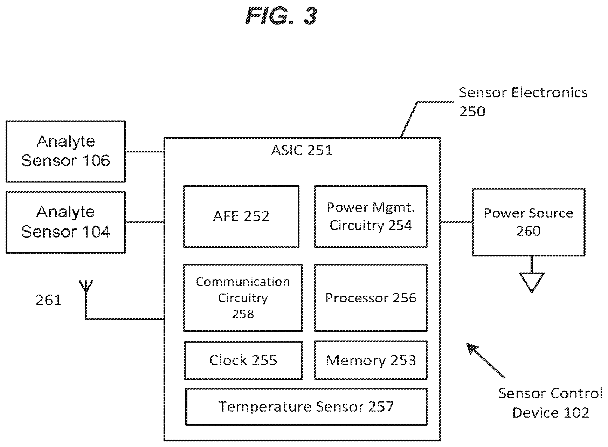

FIG. 3 is a block diagram depicting an example embodiment of sensor control device 102 having analyte sensor 104 and sensor electronics 250 (including analyte monitoring circuitry) that can have the majority of the processing capability for rendering end-result data suitable for display to the user. In FIG. 3, a single semiconductor chip 251 is depicted that may be a custom application specific integrated circuit (ASIC). Shown within ASIC 251 are certain high-level functional units, including an analog front end (AFE) 252, power management (or control) circuitry 254, processor 256, and communication circuitry 258 (which can be implemented as a transmitter, receiver, transceiver, passive circuit, or otherwise according to the communication protocol). In this embodiment, both AFE 252 and processor 256 are used as analyte monitoring circuitry, but in other embodiments either circuit can perform the analyte monitoring function. Processor 256 can include one or more processors, microprocessors, controllers, and/or microcontrollers, each of which can be a discrete chip or distributed among several different chips.

A memory 253 is also included within ASIC 251 and can be shared by the various functional units present within ASIC 251 or can be distributed amongst two or more processors. Memory 253 can also be a separate chip. Memory 253 is non-transitory and can be volatile and/or non-volatile memory. In this embodiment, ASIC 251 is coupled with power source 260, which can be a coin cell battery, or the like. AFE 252 interfaces with one or more in vivo analyte sensors 104, 106 and receives measurement data therefrom and outputs the data to processor 256 in digital form, which in turn can, in some embodiments, process in any of the manners described elsewhere herein. This data can then be provided to communication circuitry 258 for sending, by way of antenna 261, to reader device 120 (not shown), for example, where minimal further processing is needed by the resident software application to display the data. Antenna 261 can be configured according to the needs of the application and communication protocol. Antenna 261 can be, for example, a printed circuit board (PCB) trace antenna, a ceramic antenna, or a discrete metallic antenna. Antenna 261 can be configured as a monopole antenna, a dipole antenna, an F-type antenna, a loop antenna, and others.

Information may be communicated from sensor control device 102 to a second device (e.g., reader device 120) at the initiative of sensor control device 102 or reader device 120. For example, information can be communicated automatically and/or repeatedly (e.g., continuously) by sensor control device 102 when the analyte information is available, or according to a schedule (e.g., about every 1 minute, about every 5 minutes, about every 10 minutes, or the like), in which case the information can be stored or logged in a memory of sensor control device 102 for later communication. The information can be transmitted from sensor control device 102 in response to receipt of a request by the second device. This request can be an automated request, e.g., a request transmitted by the second device according to a schedule or can be a request generated at the initiative of a user (e.g., an ad hoc or manual request). In some embodiments, a manual request for data is referred to as a "scan" of sensor control device 102 or an "on-demand" data transfer from device 102. In some embodiments, the second device can transmit a polling signal or data packet to sensor control device 102, and device 102 can treat each poll (or polls occurring at certain time intervals) as a request for data and, if data is available, then can transmit such data to the second device. In many embodiments, the communication between sensor control device 102 and the second device are secure (e.g., encrypted and/or between authenticated devices), but in some embodiments the data can be transmitted from sensor control device 102 in an unsecured manner, e.g., as a broadcast to all listening devices in range.

Different types and/or forms and/or amounts of information may be sent as part of each communication including, but not limited to, one or more of current sensor measurements (e.g., the most recently obtained analyte level information temporally corresponding to the time the reading is initiated), rate of change of the measured metric over a predetermined time period, rate of the rate of change of the metric (acceleration in the rate of change), or historical metric information corresponding to metric information obtained prior to a given reading and stored in a memory of sensor control device 102.

Some or all of real time, historical, rate of change, rate of rate of change (such as acceleration or deceleration) information may be sent to reader device 120 in a given communication or transmission. In certain embodiments, the type and/or form and/or amount of information sent to reader device 120 may be preprogrammed and/or unchangeable (e.g., preset at manufacturing), or may not be preprogrammed and/or unchangeable so that it may be selectable and/or changeable in the field one or more times (e.g., by activating a switch of the system, etc.). Accordingly, in certain embodiments reader device 120 can output a current (real time) sensor-derived analyte value (e.g., in numerical format), a current rate of analyte change (e.g., in the form of an analyte rate indicator such as an arrow pointing in a direction to indicate the current rate), and analyte trend history data based on sensor readings acquired by and stored in memory of sensor control device 102 (e.g., in the form of a graphical trace). Additionally, an on-skin or sensor temperature reading or measurement may be collected by an optional temperature sensor 257. Those readings or measurements can be communicated (either individually or as an aggregated measurement over time) from sensor control device 102 to another device (e.g., reader 120). The temperature reading or measurement, however, may be used in conjunction with a software routine executed by reader device 120 to correct or compensate the analyte measurement output to the user, instead of or in addition to actually displaying the temperature measurement to the user.

In addition, although FIG. 3 depicts dual analyte sensors 104, 106, according to many embodiments of the present disclosure, sensor control device 102 can be configured to collect data indicative of multiple physiological measurements, including but not limited to, data indicative of a glucose level, lactate level, ketone level, or heart rate measurement, to name only a few. In some embodiments, for example, sensor 104 can be a dual-analyte sensor configured to sense a glucose level and a concentration of another analyte (e.g., lactate, ketone, etc.). Additional details regarding dual-analyte sensors are described, for example, in the '276 Publication referenced herein above. In some embodiments, sensor control device 102 can include multiple discrete sensors, each of which is capable of collecting data indicative of any of the aforementioned physiological measurements. For example, in some embodiments, a first analyte sensor 104 may be used to sense blood glucose, and a second analyte sensor 106 may be used to sense ketone.

### Embodiments of Systems, Devices and Methods for Combined Use of Blood Glucose and Ketone Data

Ketone Monitoring systems can provide a ketone test alert based on whether or not the most recent discrete self-monitored blood glucose (SMBG) measurement exceeds a certain pre-determined high glucose threshold. However, the connection between ketones and BG is not static. Rather, prolonged lack of insulin triggers elevation of glucagon, which in turn increases the release of glucose from the liver. In addition, the absence or extremely low levels of insulin leads to the release of free fatty acids from adipose tissue, which are converted in the liver into ketone bodies (including β-hydroxybutyrate).

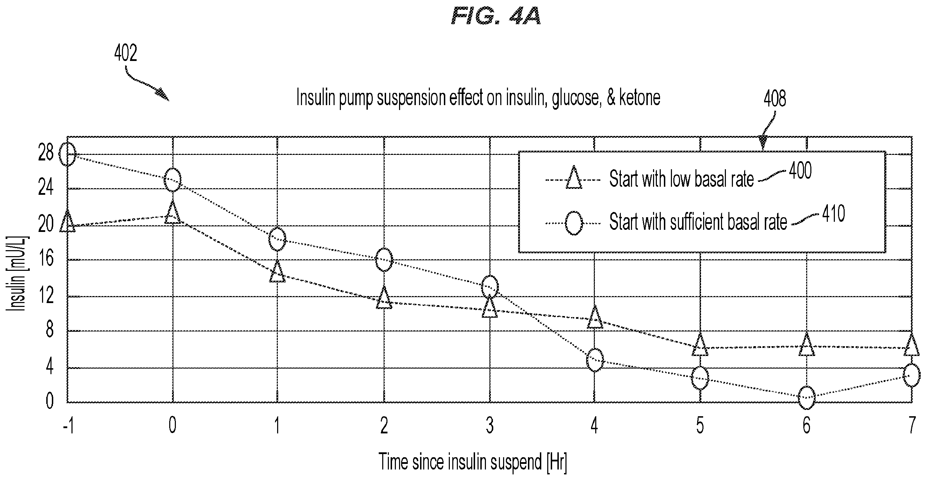

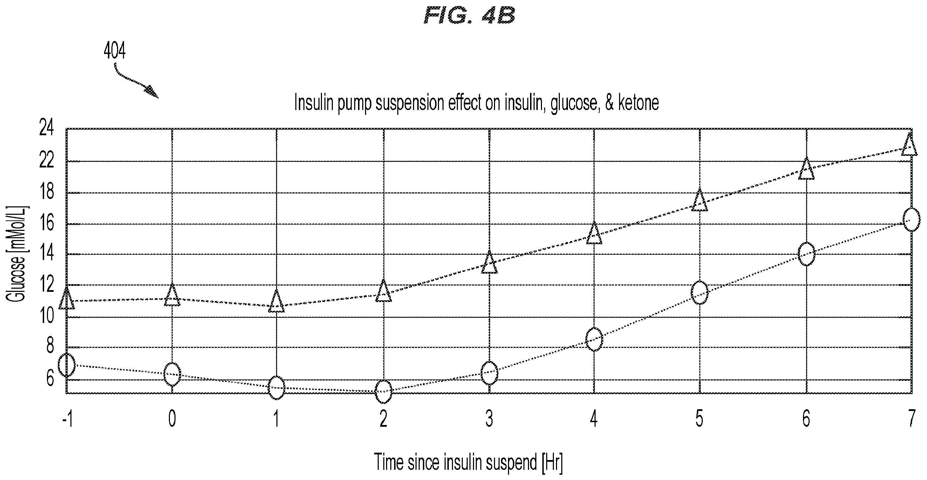

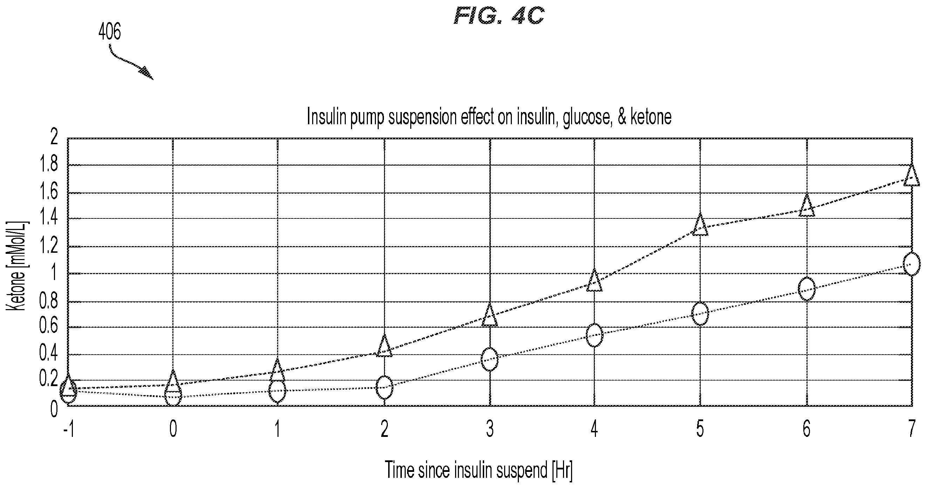

FIGS. 4A-4C illustrate an example of how ketone levels are correlated to BG levels, but do not have a static correlation to each other. The illustration is based on an insulin pump suspension study protocol [M. J. Castillo, et al., "The degree/rapidity of the metabolic deterioration following interruption of a continuous subcutaneous insulin infusion is influenced by the prevailing blood glucose Level," Journal of Clinical Endocrinology & Metabolism, vol. 81, pp. 1975-8, May 1, 1996], where study participants are individuals with T1 diabetes. The two study arms presented in FIGS. 4A-4C start with different conditions, namely with low basal rate, and with sufficient basal rate in their insulin pump systems. In the study, the insulin pump delivery is suspended, and analyte levels are measured hourly during the next several hours since pump suspension. The figures are aligned relative to the start of the suspension, marked at 0 hour. The analytes measured are insulin (FIG. 4A), glucose (FIG. 4B), and ketone (FIG. 4C) concentration levels, with a common key 408 indicating data from first and second study arms 400, 410 in all charts 401, 404, 406. Specifically, the ketone measurement chosen is β-hydroxybutyrate. Since the first study arm 400 starts with a low basal rate, the amount of insulin concentration 1 hour before and at the start of insulin pump suspension (see the insulin chart 402) is lower than that of the study arm 410 that starts with sufficient basal rate. Consequently, the glucose concentration of the first study arm 400 is higher than that of the second study arm 410, as shown in the glucose chart 404. In both study arms, as shown in the ketone chart 406, the ketone concentration levels gradually increase as the insulin pump suspension continues, as the release of free fatty acids are associated with the rise in ketone bodies. Since glucose monitoring is more common than ketone monitoring, advice to check ketone is generally based on the observation of high glucose levels (e.g. a threshold around 240 mg/dL through 250 mg/dL). For example, in the first study arm, glucose exceeding 250 mg/dL (approximately 13.9 mMol/L) occurs around 3 hours since the start of the insulin pump suspension. The corresponding ketone concentration is slightly higher than 0.6 mMol/L, just at or above values that is generally considered to be of no DKA concern. As a result, using high glucose value as a proxy for detecting DKA risk seems plausible based on data from this first study arm. However, applying the same high glucose criteria on the second study arm, glucose exceeds 250 mg/dL around 6 hours, where the ketone measurement is already at a much higher value of around 0.9 mMol/L. Using a lower glucose threshold such as 150 mg/dL (approximately 8.3 mMol/L) will catch the second study arm's ketone crossing 0.6 mMol/L promptly. However, this lower glucose threshold is already exceeded before the study for the first study arm, and thus will not be able to correctly be used to infer DKA risk.

Unlike SMBG meters, glucose sensor-based meters such as on demand systems and continuous glucose monitoring (CGM) systems have access to a longer stream of historical glucose relative to any point in time when the patient queries for glucose measurement. A model-based ketone-on-board estimation using glucose time series can significantly improve the specificity of DKA risk, and thus provide a more reliable reminder for the patient to test for ketones. Since DKA risk is directly linked to ketone history (in a concept similar to insulin-on-board), ketone test reminder based on this method can be tuned relative to point-glucose-threshold-based reminder to have an increased specificity while maintaining equal or lower false positives and false negatives.

For a manual ketone test device, the method can be used to generate a suggestion to take ketone measurement to better protect the patient from DKA risk. When the patient provides enough insulin use information (by using the built-in insulin calculator), the model can further improve the specificity of the ketone-on-board estimation.

A system for implementing the method may include two components. The first is the front end, which is related to the system user interface (UI). The second is the back end, that performs the necessary calculations for ketone-on-board estimate.

The front end provides a suggestion for the patient to take a ketone measurement whenever the back end predicts a high DKA risk based on the estimated ketone-on-board. A short explanation may be provided onscreen and/or in the user guide explaining that the recent glucose history suggests a good time to test for ketones.

The back end updates the estimated ketone-on-board model based on available glucose time series from relevant recent Scans or periodic data collection. The model may include a single glucose compartment, a single effective insulin compartment, a single plasma insulin compartment, and a single β-hydroxybutyrate compartment. Denoting glucose, effective insulin, and ketone compartments as the model states *x_{g}*, *xₑ*, and *xₖ*, the rate of change of each of these compartments at any continuous time instance *t* can be described as follows: *ẋ_{g}*(*t*) *= -*[*p*₁ ** x_{g}*(*t*)] *-* [*xₑ*(*t*) ** x_{g}*(*t*)] + [*p*₁ * *x_{NHGB}*(*t*)] *+* [*u_{M}*(*t*)], *ẋₑ*(*t*) = *-*[*p*₂ ** xₑ*(*t*)] *+* [*p*₃ ** x_{iT}*(*t*)], *ẋₖ*(*t*) = -[*p*₄ * *xₖ*(*t*)] - [*p*₅ ** f_{iT}*(*xᵢ*(*t*), *x_{iB}*(*t*))] *+* [*x_{kB}*(*t*)], *x_{iT}*(*t*) *= xᵢ*(*t*) *- x_{iB}*(*t*)*.* The additional variables and inputs are denoted as follows: *x_{NHGB}* is the rate of glucose appearance from the net hepatic glucose balance, *u_{M}* is the rate of glucose appearance from meal, *x_{iT}* is the transitory element of *xᵢ, f_{iT}* is a function describing the ketone buildup from insufficient circulating insulin, *x_{iB}* is the baseline element of *xᵢ,* and *x_{kB}* is the baseline element of *xₖ. .* The parameter *p₁* is the glucose effectiveness index that governs the rate of insulin independent glucose clearance, *p₂* is the rate of insulin clearance in the effective insulin compartment, *p₃* is the insulin activation rate constant, *p₄* is the rate of ketone clearance, and *p₅* is the accumulation rate constant due to insufficient circulating insulin. Variations of this model or other model may be used, providing a way to describe the interconnection between glucose, insulin, and ketone. Generally, the dynamic model at any continuous time instance t can be described as functions of the current compartment values represented by the states *x* and known or estimated external inputs: $\frac{d}{dt} {x}_{g} \left(t\right) : = {\dot{x}}_{g} \left(t\right) = {f}_{g} \left(t\right) , \frac{d}{dt} {x}_{e} \left(t\right) : = {\dot{x}}_{e} \left(t\right) = {f}_{e} \left(t\right) , \frac{d}{dt} {x}_{k} \left(t\right) : = {\dot{x}}_{k} \left(t\right) = {f}_{k} \left(t\right)$ , or at any sampled time instance k be described as functions of the current compartment values: *x_{g}*(*k +* 1) *= f*_{*g*2}(*k*), *xₑ*(*k +* 1) *= t*_{*e*2}(*k*), *xₖ*(*k +* 1) *= f*_{*k*2}(*k*)*.* The dynamic model is updated over time during every Scan instance or periodic time interval, where each instance computes the best estimate of these states (i.e. glucose, effective insulin, plasma insulin, and β-hydroxybutyrate compartments) as well as their variance. The main source of measurement comes from the glucose time series made available by the glucose sensor. Any closed-loop state observer forms such as the Kalman Filter may be used to reconcile measurements at each time step against the predicted values of the states, to obtain a corrected state estimation. For example, the model previously discussed can be used to generate the equations for the process model to calculate the mean estimate of the predicted state and covariance of the predicted state. Then, measurement from one or more glucose sensors can be used as the primary measurement for the measurement equation to perform the state correction or state update in the context of a Kalman Filter structure such as an Extended Kalman Filter. Whenever ketone measurement is taken by the patient, the additional information is used by the filter to refine the estimate of one or more parameters related to the ketone-on-board estimation based on glucose data. As a result, ketone-on-board is then continuously recalculated as a measure of continuous exposure to β-hydroxybutyrate levels. This could be in the form of a simple integration of β-hydroxybutyrate levels beyond a minimum threshold value, with an assumed decay rate. Whenever the exposure to β-hydroxybutyrate levels exceeds a predetermined level, the DKA risk is considered high enough to warrant an actual ketone test.

The state observer framework allows for other additional information to better improve the DKA risk estimate. In one example, for systems with built-in ketone strip compatibility, the measured ketones are used to feed back into the state observer to correct the β-hydroxybutyrate state estimate. In a second example, for systems where the patient provides insulin use information, the insulin dose and timing are used to feed back into the state observer to correct the plasma insulin state estimate. One way of obtaining this data is when the user invokes the built-in insulin calculator on a system with built-in ketone strip compatibility enabling manual testing.

In one embodiment, an improvement to high ketone alert for a person with Type 1 diabetes mellitus (T1DM) using ketone sensor is contemplated, reducing false high ketone alert not related to risk of diabetic ketoacidosis (DKA) by using information from other analyte in addition to ketone. In a single-analyte design, a high ketone threshold (which may be pre-set or be user-settable) alerts the user of high ketone to avoid the risk of DKA. In the first improved embodiment, information from a glucose sensor is used to distinguish between dangerously high ketone levels due to insufficient insulin delivery (that can lead to DKA) over high ketone levels due to successful ketone diet. The latter case is accompanied by a specific glucose pattern with very little glucose variability and very small time in high glucose. The high ketone alert will then enunciate for the first case, whereas the second case does not trigger the high ketone alert. Generally, this embodiment makes a distinction on when a threshold for one analyte (e.g. ketone) is reached, a determination is made whether the nature of the threshold crossing is a concerning one (e.g. possibility of DKA) or an encouraging one (e.g. successful ketone diet).

In a second embodiment, different types of high ketone notification/alert are created. For example, a more urgent type of enunciation is triggered for the first case described in the first embodiment, while a more positive notice is presented for the second case described in the first embodiment. In other words, the ketone alert may be modified based on the analysis of the combination of the glucose data and ketone data (for example, to distinguish between potential DKA and successful ketone diet). For example, the ketone alert may be modified by adjusting the severity of the alert, for example changing the alert types (e.g., sound instead of just vibrate), changing the volume, displaying different color alerts on the display screen, whether or not alerts are repeated and the repeat timing, etc. The alert may also include information corresponding to whether the alert is associated with the first case (e.g., potential DKA) or the second case (e.g., successful ketone diet).

In a third embodiment, the high ketone alert may be accompanied by notification to consult the patient's health care professional (HCP) when a specific situation is suspected. For example, people with T1DM may use a class of medication called SGLT-2 inhibitor (SGLT-2i). This class of medication was originally developed to manage glucose for people with T2DM, and the use of SGLT-2i on people with T1DM is on-label in some regions and off-label in others. One effect of taking SGLT-2i is the lowering of the renal clearance threshold, resulting in high glucose concentration in blood to be released in urine. As a result, it is possible for insulin-dependent cells to be deficient of glucose, triggering the process that can eventually result in DKA, while the measured glucose remains in good range, termed euglycemic DKA. To make sure that the modified alerts such as described in the first and second embodiments do not behave incorrectly when a person with T1DM is taking SGLT-2i, this third embodiment estimates whether a person is taking SGLT-2i or not by using a method as follows. When not taking SGLT-2i, a dynamic relationship can be established to estimate ketone based on glucose history. Using data from a glucose sensor and this dynamic relationship, a ketone estimate can be calculated. This ketone estimate can then be compared against the ketone reading of the ketone sensor. If a person with T1DM also takes SGLT-2i, then the estimated ketone may be consistently lower than the measured ketone. The comparison may result in concluding that the person may be taking SGLT-2i, causing the specific situation. In this case, certain high ketone alerts may have a warning or disclaimer to consult with their HCP over concomitant use of SGLT-2i. In other words, the ketone alert may be modified based on an assessment of whether the patient may be taking a medication associated with renal clearance (such as SGLT-2i).

In a fourth embodiment, the frequency of high ketone alert reasserting itself when a high threshold is reached is increased when a comparison between ketone and glucose data suggests potential DKA as opposed to ketone diet-induced high ketones.

In a fifth embodiment, the projected high ketone alert is deactivated when the suspected reason for high ketones is from ketone diet instead of potential DKA.

In a sixth embodiment, an alternate information is presented when high ketone is suspected to be caused by ketone diet, where the alert may present useful information such as number of hours achieving high ketone instead of the fact that the latest ketone exceeds a high threshold. Another alternate information could include week-to-week trend on the number of hours or percent of time achieving high ketone, day-to-day trend, or time-of-day breakdown of the trend.

In a seventh embodiment, when the use of medication that can alter the renal clearance threshold such as SGLT-2i is suspected, the use of high glucose alert is accompanied by a notification in the user interface that the lack of high glucose alert may not achieve the intended purpose, and to consult the user's HCP for guidance.

### Method For Combined Use of BG and Ketone Data

Example embodiments of methods for use of combined glucose and ketone data in operation of a medical apparatus or system will now be described. Before doing so, it will be understood by those of skill in the art that any one or more of the steps of the example methods described herein can be stored as software instructions in a non-transitory memory of a sensor control device, a reader device, a remote computer, or a trusted computer system, such as those described with respect to FIG. 1. The stored instructions, when executed, can cause the processing circuitry of the associated device or computing system to perform any one or more of the steps of the example methods described herein. It will also be understood by those of skill in the art that, in many of the embodiments, any one or more of the method steps described herein can be performed using real-time or near real-time sensor data. In other embodiments, any one or more of the method steps can be performed retrospectively with respect to stored sensor data, including sensor data from prior sensor wears by the same user. In some embodiments, the method steps described herein can be performed periodically, according to a predetermined schedule, and/or in batches of retrospective processes.

It will also be appreciated by those of skill in the art that the instructions can be stored in non-transitory memory on a single device (e.g., a sensor control device or a reader device) or, in the alternative, can be distributed across multiple discrete devices, which can be located in geographically dispersed locations (e.g., a cloud platform). For example, in some embodiments, the collection of data indicative of an analyte level (e.g., glucose, ketone) can be performed on the sensor control device, whereas the calculation of analyte metrics (e.g., glucose derivative values, ketone derivative values) and the comparison of said analyte metrics to predetermined thresholds can be performed on a reader device, remote computing system, or a trusted computing system. In some embodiments, the collection of analyte data and comparison with predetermined thresholds can be performed solely on the sensor control device. Likewise, those of skill in the art will recognize that the representations of computing devices in the embodiments disclosed herein, such as those shown in FIG. 1, are intended to cover both physical devices and virtual devices (or "virtual machines").

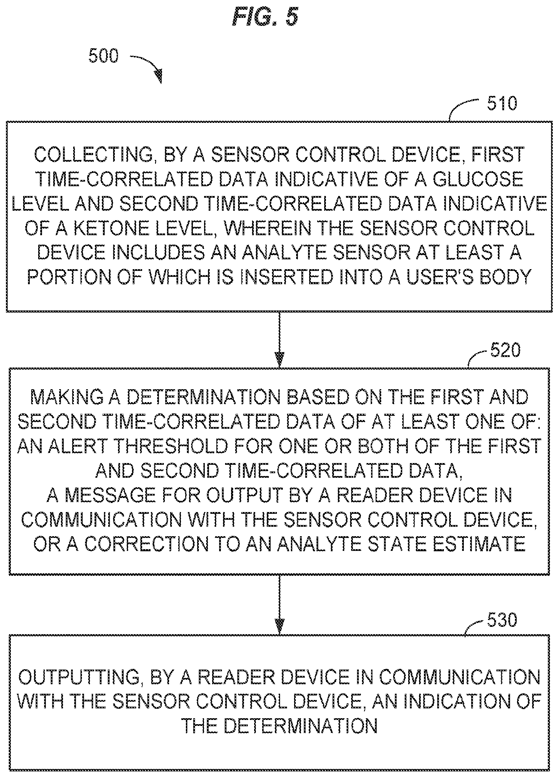

FIG. 5 is a flow diagram of an example embodiment of a method 500 for analyte monitoring. Steps of the method 500 may be performed by a system including a sensor control unit comprising an analyte sensor having a portion that is configured to be inserted into a user's body at an insertion site, wherein the portion includes a first sensing element configured to sense a glucose level in a bodily fluid and a second sensing element configured to sense an analyte indicative of a ketone level (e.g., β-hydroxybutyrate) in the bodily fluid of the same insertion site. In other embodiments, Steps 510 and 520 can be performed by a sensor control unit comprising a first analyte sensor and a second analyte sensor, wherein the first analyte sensor is configured to sense a glucose level in a bodily fluid and the second analyte sensor is configured to sense an analyte indicative of a ketone level in the bodily fluid, and wherein the first and second analyte sensors are configured to sense analyte levels at the same localized site of insertion. In another example, the analyte sensor may be a glucose sensor, and the sensor control unit may be configured to receive data indicative of a ketone level, such as from a ketone test strip.

Referring to FIG. 5, at Step 510, the method 500 may include collecting by a sensor control device first time-correlated data indicative of a glucose level and second time-correlated data indicative of a ketone level, wherein the sensor control device includes an analyte sensor at least a portion of which is inserted into a user's body. According to many of the embodiments, the first analyte metric is a glucose derivative, and the second analyte metric is a ketone derivative. At Step 520, a processor of the system, for example a processor of a reader device in communication with the sensor control device, may make a determination for controlling an output of a system component. Making the determination may be based on the first and second time-correlated data. The determination may include a determination of at least one of: an alert threshold for one or both of the first and second time-correlated data, a message for output by a reader device in communication with the sensor control device, or a correction to an analyte state estimate in a system memory. The determination may include one or more of these, and each may be provided in combination or isolation. For example, the determination may include determining an alert threshold but not determining a message for output.

Determining an alert threshold may include, for example, setting or modifying a threshold value for blood glucose, for ketone bodies, or for another analyte, which when exceed causes the reader device or another system component to output an alarm. Under nominal conditions, a high glucose alert could be set at 250mg/dL, and the reader device or another system component could be configured to output an alarm anytime data indicative of glucose level crosses this first threshold from a lower value. Similarly, a high ketone alert could be set at 1.3 mMol/L, and the reader device or another system component could be configured to output an alarm when data indicative of ketone level crosses this second threshold from a lower value. In one example, the second threshold is modified based on distinguishing between dangerously high ketone levels due to insufficient insulin delivery (which can lead to DKA) over high ketone levels due to successful ketone diet. In the former case, the second threshold is left unchanged or slightly lowered (e.g. to 1.0 mMol/L) to allow for an earlier intervention. In the latter case, the second threshold may be increased (e.g. to 1.5 mMol/L) in order to prevent false high ketone alarms. In another example, an estimate of the amount of diet-related ketone is used to adjust the second threshold, for example, by dynamically adding a fixed fraction of the estimated diet-related ketone value to the second threshold. If the latest diet-related ketone value is estimated to be 0.6 mMol/L, and the fixed fraction is set to 0.9, the second threshold is set such that the threshold is 0.9*0.6 mMol/L above the nominal value of the second threshold (previously set at 1.3 mMol/L).

Determining a message may include, for example, selecting a predetermined message from a data table in response to a result of automatic analysis of the first and second time-correlated data. Under nominal conditions, a high glucose alert could be set at 250mg/dL, and the reader device or another system component could be configured to output an alarm anytime data indicative of glucose level crosses this first threshold from a lower value. Similarly, a high ketone alert could be set at 1.3 mMol/L, and the reader device or another system component could be configured to output an alarm anytime data indicative of ketone level crosses this second threshold from a lower value. In one example, if a ketone threshold crossing relative to the second threshold occurs, and most or all of the ketone levels is estimated based on the first time-correlated data and second time-correlated data to be due to insufficient insulin delivery (that can lead to DKA), the message content may convey urgency and the need for corrective action. The enunciation of an audible alert as well as the visual aspects of the message may also correspond to a higher urgency, choosing louder audible alert, higher frequency of re enunciation when the alert is snoozed, and warmer colors on the message. In another example, if a ketone threshold crossing relative to the second threshold occurs, and none or a very small amount of the ketone level is estimated based on the first time-correlated data and second time-corelated data to be due to insufficient insulin delivery (that can lead to DKA), the message content may be congratulatory and may not assert itself over more urgent messages such as impending hypoglycemia or hyperglycemia. In another example, if a ketone threshold crossing relative to the second threshold occurs, and none or a very small amount of the ketone levels is estimated based on the first time-correlated data and second time-correlated data to be due to successful ketone diet, the message content may convey urgency and the need for corrective action. The enunciation of an audible alert as well as the visual aspects of the message may also correspond to a higher urgency, choosing louder audible alert, higher frequency of re enunciation when the alert is snoozed, and warmer colors on the message. In another example, if a ketone threshold crossing relative to the second threshold occurs, and most or all of the amount of the ketone level is estimated based on the first time-correlated data and second time-corelated data to be due to successful ketone diet, the message content may be congratulatory and may not assert itself over more urgent messages such as impending hypoglycemia or hyperglycemia.

Determining a correction to an analyte state estimate may include, for example, calculating a correction factor for, or corrected value of, an initial estimate for blood glucose or other analyte made based on the first time-correlated data only. For example, the time-correlated data indicative of a glucose level could contain a slowly varying error. Prior to calculating the correction factor, the estimated states related to glucose may also be affected by this error. Instead, a correction factor can be applied to adjust the states related to glucose in order to improve the estimated blood glucose value.

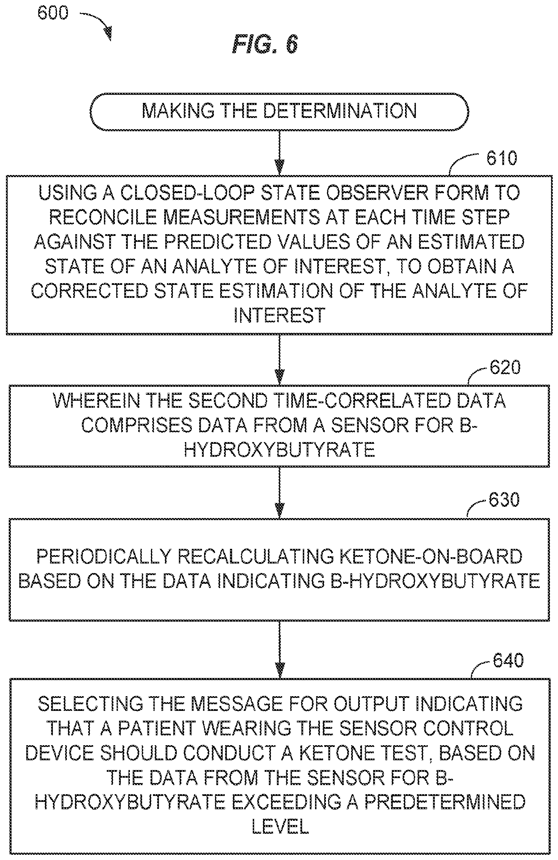

A more detailed description of these determinations and related operations are provided in connection with FIGS. 6-10 and herein above.

At Step 530, the method 500 may include outputting, by a reader device in communication with the sensor control device, an indication of the determination. For example, the reader device may output an audible and/or visible alarm, display an alarm message, display an informational message, or correct one or more analyte values held in memory and set an indicator indicating the value is corrected. In some embodiments, generation of an alert indication may include outputting a notification or message for display by user's mobile device running a reader application. In an alternative, or in addition, an alert indication may include one or more of a visual, audio, or vibratory alert or alarm that is output to a display of a reader device, remote computer, or trusted computer system. A remedial action can be optionally performed in response to, or instead of, an alarm. In some embodiments, for example, the remedial action can be suppressing or modifying indication of a low glucose alarm. In other embodiments, a remedial action may include preventing the issuance of a command to alter or cause the delivery of medication (e.g., insulin) by an automated medication delivery system (e.g., insulin pump).

In some embodiments, making the determination at Step 520 of the method 500 may include certain additional operations 600 as shown in FIG. 6. At Step 610, making the determination may include using a closed-loop state observer form to reconcile measurements at each time step against the predicted values of an estimated state of an analyte of interest, to obtain a corrected state estimation of the analyte of interest. One example of a closed-loop state observer is a Kalman Filter or variations thereof. The process model and the measurement model may be constructed based on a dynamic model involving available measurements, such as glucose, effective insulin, and ketones. There can be model states that correspond to these quantities, denoted by *x_{g}, xₑ,* and *xₖ* for glucose, effective insulin, and ketone-related states, respectively. Generally, the dynamic model at any continuous time instance t can be described as functions of the current compartment values x and known or estimated external inputs: $\frac{d}{dt} {x}_{g} \left(t\right) : = {\dot{x}}_{g} \left(t\right) = {f}_{g} \left(t\right) , \frac{d}{dt} {x}_{e} \left(t\right) : = {\dot{x}}_{e} \left(t\right) = {f}_{e} \left(t\right) , \frac{d}{dt} {x}_{k} \left(t\right) : = {\dot{x}}_{k} \left(t\right) = {f}_{k} \left(t\right)$ , or at any sampled time instance k be described as functions of the current compartment values: *x_{g}*(*k +* 1) *= f*_{*g*2}(*k*), *xₑ*(*k +* 1) *= f*_{*e*2}(*k*), *xₖ*(*k +* 1) = *f*_{*k*2}(*k*)*.* In each time step, the process model will predict the value of the latest states that can include *x_{g}, xₑ,* or *xₖ.* The available measurements are then used by the closed loop state observer (such as a Kalman Filter) to obtain a corrected state estimation of the analyte of interest. In an aspect of the operations 600, at 620, the second time-correlated data may be, or may include, data from a sensor for β-hydroxybutyrate. At step 630, making the determination may further include periodically recalculating ketone-on-board based on the sensor data indicating β-hydroxybutyrate, using a known correlation factor. Periodic recalculation may be performed by a processor of a reader device at each time step, for example, once per tenth of a second, once per second, once per ten seconds, once per minute, etc. In some embodiments, frequent measurements of β-hydroxybutyrate may not be available, and the corrector of the closed-loop state observer may only have access to ketone measurements when a ketone test strip data is used. At step 640, making the determination may further include selecting a message for output indicating that a patient wearing the sensor control device should conduct a ketone test, based on the value of the ketone-related state exceeding a predetermined level. Returning to FIG. 4, this means that the ketone-related state replaces frequent β-hydroxybutyrate measurements, and the threshold can be identical to the threshold value used if frequent β-hydroxybutyrate measurements were available, or a certain confidence interval of crossing the threshold is used to determine when a message for output indicating that a patient wearing the sensor control device should conduct a ketone test is asserted.

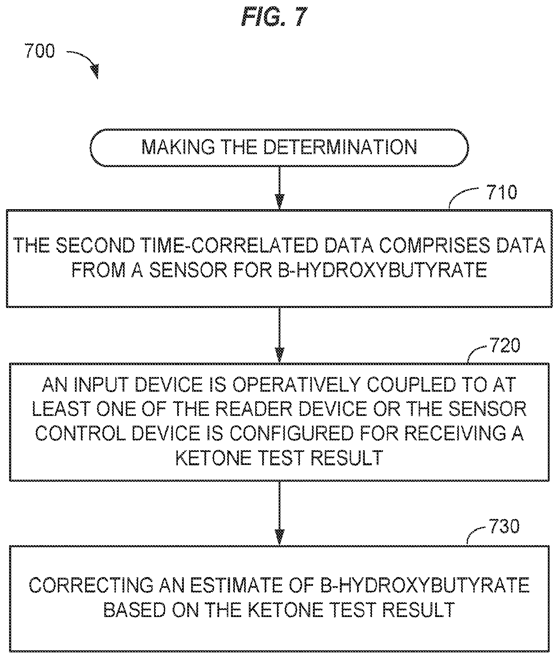

In other embodiments, making the determination at Step 520 of the method 500 may include certain additional operations and aspects 700 as shown in FIG. 7. At 710, the second time-correlated data may be, or may include, data from a sensor for β-hydroxybutyrate. In this embodiment, as indicated at 720, an input device is operatively coupled to at least one of the reader device or the sensor control device and configured for receiving a ketone test result. For example, a sensor control device may be configured with a reader for a ketone test strip. At step 720, making the determination further includes correcting an estimate of β-hydroxybutyrate based on the ketone test result. For example, using a closed-loop state observer like a Kalman Filter, the state correction process when the additional ketone test result is available can be done using the standard Kalman Filter framework.

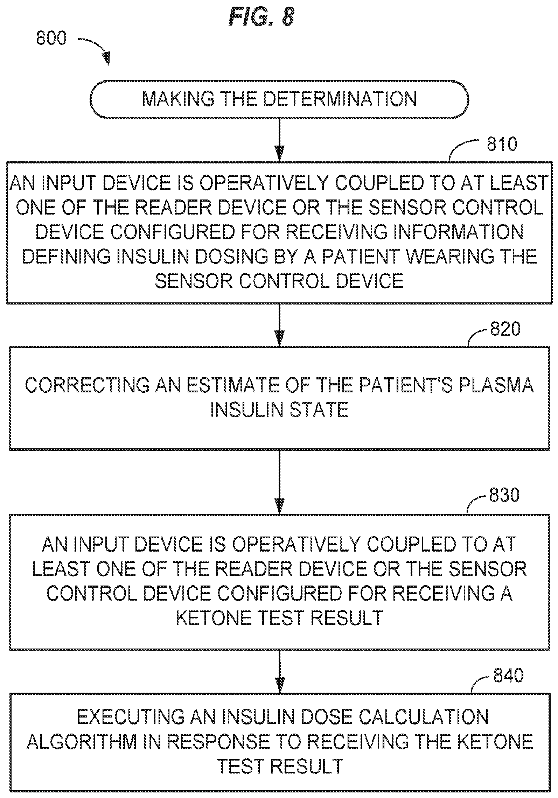

In other embodiments, making the determination at Step 520 of the method 500 may include certain additional operations 800 as shown in FIG. 8. In these embodiments, as indicated at 810, an input device may be operatively coupled to at least one of the reader device or the sensor control device configured for receiving information defining insulin dosing by a patient wearing the sensor control device. For example, the reader device may prompt the user to enter insulin dosing information at certain times, or in response to user input. At step 820, the method 500 may further include correcting an estimate of the patient's plasma insulin state. For example, using a closed-loop state observer like a Kalman Filter, the state correction process when the additional insulin state is available can be done using the standard Kalman Filter framework. Practically, the patient's plasma insulin state can be in the form of an insulin type and amount. The amount can be in terms of a delivery rate from an insulin delivery device (e.g. insulin pump) or in the form of an insulin bolus dose from an insulin delivery device (e.g. insulin pump or connected insulin pen).

In an alternative, or in addition, as indicated at 830, an input device may be operatively coupled to at least one of the reader device or the sensor control device configured for receiving a ketone test result. For example, a sensor control device may be configured with a reader for a ketone test strip, or a reader device may prompt the user to enter a ketone test result. At Step 840, the method 500 may include automatically executing an insulin dose calculation algorithm in response to receiving the ketone test result.

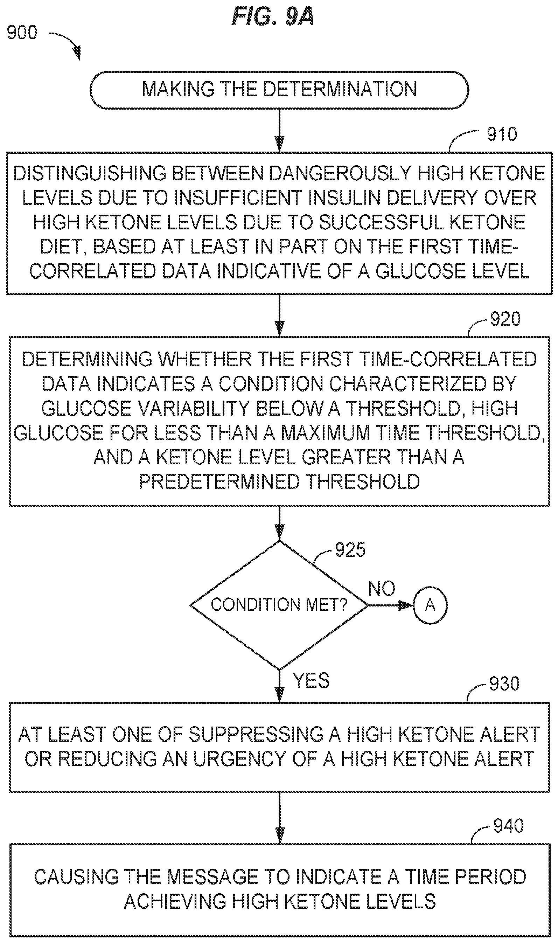

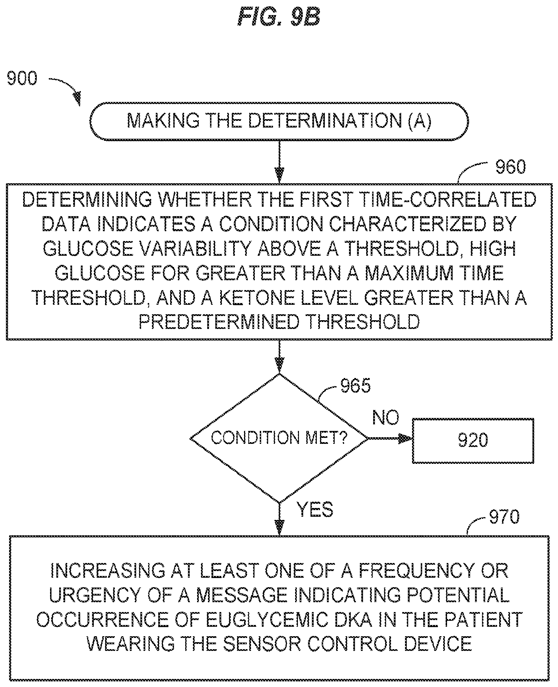

In other embodiments, making the determination at Step 520 of the method 500 may include certain additional operations 900 as shown in FIGS. 9A-9B. The operations may include, at Step 910, distinguishing between dangerously high ketone levels due to insufficient insulin delivery over high ketone levels due to successful ketone diet, based at least in part on the first time-correlated data indicative of a glucose level. For example, at Step 920, a reader device may determine whether the first time-correlated data indicates a condition characterized by glucose variability below a threshold, high glucose for less than a maximum time threshold, and a ketone level greater than a predetermined threshold. At Step 925, if the condition is detected, the system may perform at least one of suppressing a high ketone alert or reducing an urgency of a high ketone alert. For example, at Step 930 the system may suppress a high ketone alert until the indicated condition is no longer satisfied, or may accompany the alert with a notice indicating that the ketone condition is not indicative or an urgent condition, or may indicate ketosis caused by a low-carbohydrate diet. In an alternative, or in addition, at Step 940 the system may output a message of interest to a user interested in achieving intentional dietary ketosis, for example, causing the message to indicate a time period achieving high ketone levels, i.e., how long the ketosis state has lasted. Generally, this embodiment makes a distinction on when a threshold for one analyte (e.g. ketone) is reached, a determination is made whether the nature of the threshold crossing is a concerning one (e.g. possibility of DKA) or an encouraging one (e.g. successful ketone diet). Depending on the determination, the content of the messaging (i.e. what is communicated to the user, in terms of concerning information or encouraging information) and the timing of the messaging (i.e. whether it is enunciated upon triggering or enunciated at pre-determined notification times, whether the enunciation periodically re-emerges until the condition disappears or whether the enunciation occurs only at the time of triggering) can vary.

In an alternative, or in addition, if the system determines at Step 925 that the condition is not satisfied or if the system does not perform the testing Step 920, the system may at Step 960 determine whether the first time-correlated data indicates a condition characterized by glucose variability above a threshold, high glucose for greater than a maximum time threshold, and a ketone level greater than a predetermined threshold. If at 965 the system determines the condition is met, at Step 970 the system may increase at least one of a frequency or urgency of a message indicating potential occurrence of euglycemic DKA in the patient wearing the sensor control device.

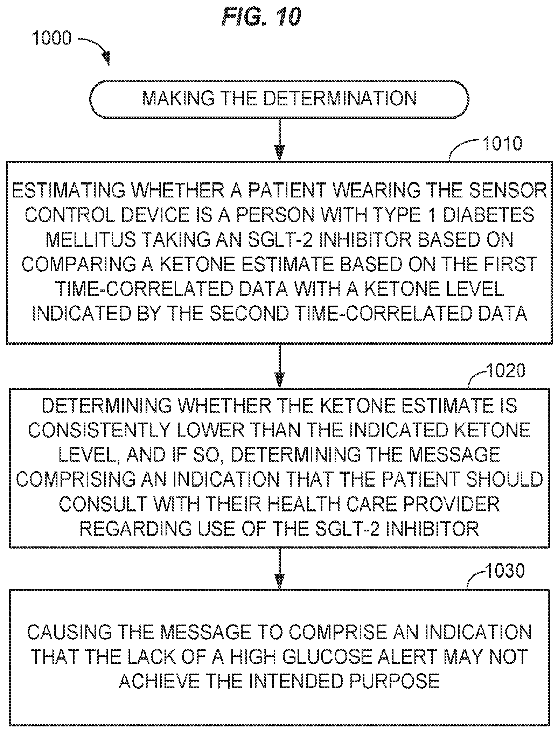

In other embodiments, making the determination at Step 520 of the method 500 may include certain additional operations 1000 as shown in FIG. 10. At step 1010, the method 500 may include, by a system processor, estimating whether a patient wearing the sensor control device is a person with Type 1 diabetes mellitus taking an SGLT-2 inhibitor based on comparing a ketone estimate based on the first time-correlated data with a ketone level indicated by the second time-correlated data. At Step 1020, the method may include determining whether the ketone estimate is consistently lower than the indicated ketone level, and if so, determining the message comprising an indication that the patient should consult with their health care provider regarding use of the SGLT-2 inhibitor. In addition, or in an alternative, at Step 1030 the method 500 may include causing the message to include an indication that the lack of a high glucose alert may not achieve the intended purpose of glucose monitoring.

Additionally, in some embodiments, any of the method steps described herein, including but not limited to the making of the determination (Step 520) and/or the outputting an indication of the determination (Step 530) can be performed on a remote monitoring device, or a cloud-based server that is communicatively coupled with a remote monitoring device. In some embodiments, the remote monitoring device can comprise, for example, a secondary reader device (e.g., a second smart phone) that is configured to be used by a third-party caretaker (e.g., the parent of a child wearing a sensor, the adult child of an elderly parent wearing a sensor, or a health care professional responsible for monitoring a patient wearing a sensor). In many embodiments, the secondary reader device can include one or more processors coupled with a memory for storing a remote analyte monitoring program that is configured to perform any one or more of the method steps described herein. For example, in certain embodiments, the remote analyte monitoring program on the secondary reader device can output an audible and/or visible alarm, display an alarm message, display an informational message, or correct one or more analyte values held in memory set and an indicator indicating the value is corrected. In some embodiments, the generation of an alert indication may include outputting a notification or message for display by the secondary reader device running the remote analyte monitoring program. In an alternative, or in addition, an alert indication may include one or more of a visual, audio, or vibratory alert or alarm that is output to a display of the secondary reader device. According to another aspect of some embodiments, the remote analyte monitoring program can permit the caretaker to configure their own alarm settings such as, e.g., enabling or disabling certain alarms, or changing certain analyte thresholds. Additional details of remote analyte monitoring programs are described in the following publications, which are hereby incorporated by reference for all purposes in their entireties: U.S. Publ. No. 2022/0248988 and U.S. Publ. No. 2022/0240819.

For each and every embodiment of a method disclosed herein, systems and devices capable of performing each of those embodiments are covered within the scope of the present disclosure. For example, embodiments of sensor control devices are disclosed and these devices can have one or more analyte sensors, analyte monitoring circuits (e.g., an analog circuit), memories (e.g., for storing instructions), power sources, communication circuits, transmitters, receivers, clocks, counters, times, temperature sensors, processors (e.g., for executing instructions) that can perform any and all method steps or facilitate the execution of any and all method steps. These sensor control device embodiments can be used and can be capable of use to implement those steps performed by a sensor control device from any and all of the methods described herein. Similarly, embodiments of reader devices are disclosed, and these devices can have one or more memories (e.g., for storing instructions), power sources, communication circuits, transmitters, receivers, clocks, counters, times, and processors (e.g., for executing instructions) that can perform any and all method steps or facilitate the execution of any and all method steps. These reader device embodiments can be used and can be capable of use to implement those steps performed by a reader device from any and all of the methods described herein. Embodiments of computer devices and servers are disclosed, and these devices can have one or more memories (e.g., for storing instructions), power sources, communication circuits, transmitters, receivers, clocks, counters, times, and processors (e.g., for executing instructions) that can perform any and all method steps or facilitate the execution of any and all method steps. These reader device embodiments can be used and can be capable of use to implement those steps performed by a reader device from any and all of the methods described herein.

Computer program instructions for carrying out operations in accordance with the described subject matter may be written in any combination of one or more programming languages, including an object-oriented programming language such as Java, JavaScript, Smalltalk, C++, C#, Transact-SQL, XML, PHP or the like and conventional procedural programming languages, such as the "C" programming language or similar programming languages. The program instructions may execute entirely on the user's computing device, partly on the user's computing device, as a stand-alone software package, partly on the user's computing device and partly on a remote computing device or entirely on the remote computing device or server. In the latter scenario, the remote computing device may be connected to the user's computing device through any type of network, including a local area network (LAN) or a wide area network (WAN), or the connection may be made to an external computer (for example, through the Internet using an Internet Service Provider).

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the foregoing description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art. Aspects of the invention are set out in the independent claims. Preferred features are set out in the dependent claims and may be implemented in combination together with each of the aspects set out in the independent claims. Apparatus comprising means for implementing each of the methods are also provided. Features of one aspect may be applied to each aspect alone or in combination with other features.

To the extent the embodiments disclosed herein include or operate in association with memory, storage, and/or computer readable media, then that memory, storage, and/or computer readable media are non-transitory. Accordingly, to the extent that memory, storage, and/or computer readable media are covered by one or more claims, then that memory, storage, and/or computer readable media is only non-transitory.

As used herein and in the appended claims, the singular forms "a," "an," and "the" include plural referents unless the context clearly dictates otherwise.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

Systems, devices, and methods for a dual analyte sensor using glucose history from a glucose sensor in combination with data from a ketone sensor to control operation of a user interface device or insulin pump are provided. In some embodiments, the systems, apparatus or methods may make use of combination of glucose history and a β-hydroxybutyrate physiological model to better predict diabetic ketoacidosis (DKA), in comparison to a prediction based on a simple high glucose threshold. In other embodiments, the systems, apparatus or method may include features for generating an estimate of the patient's medication state and/or knowledge of medication information, such as a patient with T1 diabetes mellitus (DM) using an SGLT-2 inhibitor.

The disclosure of this application also contains the following numbered clauses:
1. An analyte monitoring system, comprising:
   a sensor control device including an analyte sensor, wherein the analyte sensor includes at least a portion configured to be inserted into a user's body, wherein the sensor control device is configured to collect first time-correlated data indicative of a glucose level and second time-correlated data indicative of a ketone level, and wherein the sensor control device is operatively coupled to a first processing circuitry, and a first non-transitory memory; and
   a reader device comprising second processing circuitry and a second non-transitory memory,
   wherein at least one of the first or the second non-transitory memory includes instructions that, when executed, cause at least one of the first or the second processing circuitry to perform:
      making a determination based on the first and second time-correlated data of at least one of:
         an alert threshold for one or both of the first and second time-correlated data,
         a message for output by the reader device, or
         a correction to an analyte state estimate; and
      outputting, by the reader device, an indication of the determination.
2. The analyte monitoring system of clause 1, wherein the instructions for making the determination further cause using a closed-loop state observer form to reconcile measurements at each time step against the predicted values of an estimated state of an analyte of interest, to obtain a corrected state estimation of the analyte of interest.
3. The analyte monitoring system of clause 1 or 2, wherein the second time-correlated data comprises data from a ketone sensor.
4. The analyte monitoring system of clause 3, wherein the second time-correlated data comprises data indicating β-hydroxybutyrate.
5. The analyte monitoring system clause 4, wherein the instructions for making the determination further cause periodically recalculating ketone-on-board based on the data indicating β-hydroxybutyrate.
6. The analyte monitoring system of clause 4 or 5, wherein the instructions for making the determination cause selecting the message for output indicating that a patient wearing the sensor control device should conduct a ketone test, based on the data from the sensor for β-hydroxybutyrate exceeding a predetermined level.
7. The analyte monitoring system of any preceding clause, wherein the second time-correlated data comprises data from a sensor for β-hydroxybutyrate, and further comprising an input device operatively coupled to at least one of the reader device or the sensor control device configured for receiving a ketone test result, wherein the instructions for making the determination cause correcting an estimate of β-hydroxybutyrate based on the ketone test result.
8. The analyte monitoring system of any preceding clause, further comprising an input device operatively coupled to at least one of the reader device or the sensor control device configured for receiving information defining insulin dosing by a patient wearing the sensor control device, wherein the instructions for making the determination cause correcting an estimate of the patient's plasma insulin state.
9. The analyte monitoring system of any preceding clause, further comprising an input device operatively coupled to at least one of the reader device or the sensor control device configured for receiving a ketone test result, wherein the instructions further cause automatic execution of an insulin dose calculation algorithm in response to receiving the ketone test result.
10. The analyte monitoring system of any preceding clause, wherein the indication of the determination is an alert condition for one analyte, and wherein the alert condition is based on more than one analyte.
11. The analyte monitoring system of clause 10, wherein the instructions for the distinguishing cause determining whether the first time-correlated data indicates a condition comprising glucose variability below a threshold, high glucose for less than a maximum time threshold, and a ketone level greater than a predetermined threshold.
12. The analyte monitoring system of clause 11, wherein the instructions for the determining cause at least one of suppressing a high ketone alert or reducing an urgency of a high ketone alert.
13. The analyte monitoring system of clause 11 or 12, wherein the instructions for the determining cause the message to indicate a time period achieving high ketone levels.
14. The analyte monitoring system of any of clauses 10 to 13, wherein the instructions for the distinguishing cause determining whether the first time-correlated data indicates a condition comprising glucose variability above a threshold, high glucose for greater than a maximum time threshold, and a ketone level greater than a predetermined threshold, and if the condition is detected, performing increasing at least one of a frequency or urgency of a message indicating potential occurrence of euglycemic DKA in the patient wearing the sensor control device.
15. The analyte monitoring system of any preceding clause, wherein the instructions for making the determination further cause estimating whether a patient wearing the sensor control device is a person with Type 1 diabetes mellitus taking an SGLT-2 inhibitor based on comparing a ketone estimate based on the first time-correlated data with a ketone level indicated by the second time-correlated data.
16. The analyte monitoring system of clause 15, wherein the instructions for making the determination cause determining whether the ketone estimate is consistently lower than the indicated ketone level, and if so, determining the message comprising an indication that the patient should consult with their health care provider regarding use of the SGLT-2 inhibitor.
17. The analyte monitoring system of clause 16, wherein the instructions for making the determination further cause determining the message comprising an indication that the lack of a high glucose alert may not achieve the intended purpose.
18. The analyte monitoring system of any preceding clause, wherein the instructions are stored on the second non-transitory memory.
19. The analyte monitoring system of any preceding clause, wherein the instructions are stored on the first non-transitory memory.
20. The analyte monitoring system of any preceding clause, wherein the sensor control device further includes wireless communications circuitry configured to transmit the first and the second data to the reader device.
21. The analyte monitoring system of clause 20, wherein the wireless communications circuitry is configured to transmit the first and the second data according to a Bluetooth protocol.
22. The analyte monitoring system of any preceding clause, wherein the analyte sensor is a first analyte sensor, wherein the sensor control device further includes a second analyte sensor, wherein the first analyte sensor is configured to sense a glucose level in a bodily fluid, and wherein the second analyte sensor is configured to sense a ketone level in the bodily fluid.
23. The analyte monitoring system of any preceding clause, further comprising a medication delivery device.
24. The analyte monitoring system of clause 23, wherein the medication delivery device comprises an insulin pump.
25. A computer-implemented method for detecting a suspected glucose dropout, the method comprising:
   collecting, by a sensor control device, first time-correlated data indicative of a glucose level and second time-correlated data indicative of a ketone level, wherein the sensor control device includes an analyte sensor at least a portion of which is inserted into a user's body;
   making a determination based on the first and second time-correlated data of at least one of:
      an alert threshold for one or both of the first and second time-correlated data,
      a message for output by a reader device in communication with the sensor control device, or
      a correction to an analyte state estimate; and
   outputting, by a reader device in communication with the sensor control device, an indication of the determination.
26. The method of clause 25, wherein making the determination further comprises using a closed-loop state observer form to reconcile measurements at each time step against the predicted values of an estimated state of an analyte of interest, to obtain a corrected state estimation of the analyte of interest.
27. The method of clause 24 or 25, wherein the second time-correlated data comprises data from a glucose sensor.
28. The analyte monitoring system of clause 27, wherein the second time-correlated data comprises data indicating β-hydroxybutyrate.
29. The method of clause 28, wherein making the determination further comprises periodically recalculating ketone-on-board based on the data indicating β-hydroxybutyrate.
30. The method of clause 28 or 29, wherein making the determination further comprises selecting the message for output indicating that a patient wearing the sensor control device should conduct a ketone test, based on the data from the sensor for β-hydroxybutyrate exceeding a predetermined level.
31. The method of any of clauses 25 to 29, wherein the second time-correlated data comprises data from a sensor for β-hydroxybutyrate, wherein an input device is operatively coupled to at least one of the reader device or the sensor control device is configured for receiving a ketone test result, wherein making the determination further comprises correcting an estimate of β-hydroxybutyrate based on the ketone test result.
32. The method of any preceding clause, wherein an input device is operatively coupled to at least one of the reader device or the sensor control device configured for receiving information defining insulin dosing by a patient wearing the sensor control device, and making the determination further comprises correcting an estimate of the patient's plasma insulin state.
33. The method of any preceding clause, wherein an input device is operatively coupled to at least one of the reader device or the sensor control device configured for receiving a ketone test result, further comprising automatically executing an insulin dose calculation algorithm in response to receiving the ketone test result.
34. The method of any preceding clause, wherein the indication of the determination is an alert condition for one analyte, and wherein the alert condition is based on more than one analyte.
35. The method of clause 34, wherein the distinguishing further comprises determining whether the first time-correlated data indicates a condition characterized by glucose variability below a threshold, high glucose for less than a maximum time threshold, and a ketone level greater than a predetermined threshold.
36. The method of clause 35, wherein the determining comprises at least one of suppressing a high ketone alert or reducing an urgency of a high ketone alert.
37. The method of clause 35 or 36, wherein the determining comprises causing the message to indicate a time period achieving high ketone levels.
38. The method of any of clauses 34 to 37, wherein the distinguishing comprises determining whether the first time-correlated data indicates a condition characterized by glucose variability above a threshold, high glucose for greater than a maximum time threshold, and a ketone level greater than a predetermined threshold, and if the condition is detected, performing increasing at least one of a frequency or urgency of a message indicating potential occurrence of euglycemic DKA in the patient wearing the sensor control device.
39. The method of any preceding clause, wherein making the determination further comprises estimating whether a patient wearing the sensor control device is a person with Type 1 diabetes mellitus taking an SGLT-2 inhibitor based on comparing a ketone estimate based on the first time-correlated data with a ketone level indicated by the second time-correlated data.
40. The method of clause 39, wherein making the determination comprises determining whether the ketone estimate is consistently lower than the indicated ketone level, and if so, determining the message comprising an indication that the patient should consult with their health care provider regarding use of the SGLT-2 inhibitor.
41. The method of clause 40, wherein making the determination further causing the message to comprise an indication that the lack of a high glucose alert may not achieve the intended purpose.
42. The method of any preceding clause, further comprising wirelessly transmitting the first and the second data from the sensor control device to the reader device.
43. A computer program, computer program product, or computer readable medium comprising instructions which, when executed by a processor, cause the processor to perform the method of any of clauses 25 to 42.
44. An apparatus comprising:
   means for collecting, by a sensor control device, first time-correlated data indicative of a glucose level and second time-correlated data indicative of a ketone level, wherein the sensor control device includes an analyte sensor at least a portion of which is inserted into a user's body;
   means for making a determination based on the first and second time-correlated data of at least one of:
      an alert threshold for one or both of the first and second time-correlated data,
      a message for output by a reader device in communication with the sensor control device, or
      a correction to an analyte state estimate; and
   means for outputting, by a reader device in communication with the sensor control device, an indication of the determination.
45. The apparatus of clause 44, further comprising means for implementing the method of any of clauses 25 to 42.

The claims of the parent application are reproduced below. These clauses define preferable combinations of features. The applicant reserves the right to pursue protection for these combinations of features, and/or any other subject-matter contained in the parent application as filed, either in the present divisional application or in a further application divided from the present divisional application. The claims of the parent application are not the claims of the current application which are contained in a separate section headed "claims".
1. An analyte monitoring system, comprising:
   a sensor control device including an analyte sensor, wherein the analyte sensor includes at least a portion configured to be inserted into a user's body, wherein the sensor control device is configured to collect first time-correlated data indicative of a glucose level and second time-correlated data indicative of a ketone level, and wherein the sensor control device is operatively coupled to a first processing circuitry, and a first non-transitory memory; and
   a reader device comprising second processing circuitry and a second non-transitory memory,
   wherein at least one of the first or the second non-transitory memory includes instructions that, when executed, cause at least one of the first or the second processing circuitry to perform:
      making a determination based on the first and second time-correlated data of at least one of:
         an alert threshold for one or both of the first and second time-correlated data,
         a message for output by the reader device, or
         a correction to an analyte state estimate; and
      outputting, by the reader device, an indication of the determination.
2. The analyte monitoring system of claim 1, wherein the instructions for making the determination further cause using a closed-loop state observer form to reconcile measurements at each time step against the predicted values of an estimated state of an analyte of interest, to obtain a corrected state estimation of the analyte of interest.
3. The analyte monitoring system of claim 2, wherein the second time-correlated data comprises data from a ketone sensor.
4. The analyte monitoring system of claim 3, wherein the second time-correlated data comprises data indicating β-hydroxybutyrate.
5. The analyte monitoring system of claim 4, wherein the instructions for making the determination further cause periodically recalculating ketone-on-board based on the data indicating β-hydroxybutyrate.
6. The analyte monitoring system of claim 4, wherein the instructions for making the determination cause selecting the message for output indicating that a patient wearing the sensor control device should conduct a ketone test, based on the data from the sensor for β-hydroxybutyrate exceeding a predetermined level.
7. The analyte monitoring system of claim 1, wherein the second time-correlated data comprises data from a sensor for β-hydroxybutyrate, and further comprising an input device operatively coupled to at least one of the reader device or the sensor control device configured for receiving a ketone test result, wherein the instructions for making the determination cause correcting an estimate of β-hydroxybutyrate based on the ketone test result.
8. The analyte monitoring system of claim 1, further comprising an input device operatively coupled to at least one of the reader device or the sensor control device configured for receiving information defining insulin dosing by a patient wearing the sensor control device, wherein the instructions for making the determination cause correcting an estimate of the patient's plasma insulin state.
9. The analyte monitoring system of claim 8, further comprising an input device operatively coupled to at least one of the reader device or the sensor control device configured for receiving a ketone test result, wherein the instructions further cause automatic execution of an insulin dose calculation algorithm in response to receiving the ketone test result.
10. The analyte monitoring system of claim 1, wherein the indication of the determination is an alert condition for one analyte, and wherein the alert condition is based on more than one analyte.
11. The analyte monitoring system of claim 10, wherein the instructions for the distinguishing cause determining whether the first time-correlated data indicates a condition comprising glucose variability below a threshold, high glucose for less than a maximum time threshold, and a ketone level greater than a predetermined threshold.
12. The analyte monitoring system of claim 11, wherein the instructions for the determining cause at least one of suppressing a high ketone alert or reducing an urgency of a high ketone alert.
13. The analyte monitoring system of claim 11, wherein the instructions for the determining cause the message to indicate a time period achieving high ketone levels.
14. The analyte monitoring system of claim 10, wherein the instructions for the distinguishing cause determining whether the first time-correlated data indicates a condition comprising glucose variability above a threshold, high glucose for greater than a maximum time threshold, and a ketone level greater than a predetermined threshold, and if the condition is detected, performing increasing at least one of a frequency or urgency of a message indicating potential occurrence of euglycemic DKA in the patient wearing the sensor control device.
15. The analyte monitoring system of claim 1, wherein the instructions for making the determination further cause estimating whether a patient wearing the sensor control device is a person with Type 1 diabetes mellitus taking an SGLT-2 inhibitor based on comparing a ketone estimate based on the first time-correlated data with a ketone level indicated by the second time-correlated data.
16. The analyte monitoring system of claim 15, wherein the instructions for making the determination cause determining whether the ketone estimate is consistently lower than the indicated ketone level, and if so, determining the message comprising an indication that the patient should consult with their health care provider regarding use of the SGLT-2 inhibitor.
17. The analyte monitoring system of claim 16, wherein the instructions for making the determination further cause determining the message comprising an indication that the lack of a high glucose alert may not achieve the intended purpose.
18. The analyte monitoring system of claim 1, wherein the instructions are stored on the second non-transitory memory.
19. The analyte monitoring system of claim 1, wherein the instructions are stored on the first non-transitory memory.
20. The analyte monitoring system of claim 1, wherein the sensor control device further includes wireless communications circuitry configured to transmit the first and the second data to the reader device.
21. The analyte monitoring system of claim 20, wherein the wireless communications circuitry is configured to transmit the first and the second data according to a Bluetooth protocol.
22. The analyte monitoring system of claim 1, wherein the analyte sensor is a first analyte sensor, wherein the sensor control device further includes a second analyte sensor, wherein the first analyte sensor is configured to sense a glucose level in a bodily fluid, and wherein the second analyte sensor is configured to sense a ketone level in the bodily fluid.
23. The analyte monitoring system of claim 1, further comprising a medication delivery device.
24. The analyte monitoring system of claim 23, wherein the medication delivery device comprises an insulin pump.
25. A computer-implemented method for detecting a suspected glucose dropout, the method comprising:
   collecting, by a sensor control device, first time-correlated data indicative of a glucose level and second time-correlated data indicative of a ketone level, wherein the sensor control device includes an analyte sensor at least a portion of which is inserted into a user's body;
   making a determination based on the first and second time-correlated data of at least one of:
      an alert threshold for one or both of the first and second time-correlated data,
      a message for output by a reader device in communication with the sensor control device, or
      a correction to an analyte state estimate; and
   outputting, by a reader device in communication with the sensor control device, an indication of the determination.
26. The method of claim 25, wherein making the determination further comprises using a closed-loop state observer form to reconcile measurements at each time step against the predicted values of an estimated state of an analyte of interest, to obtain a corrected state estimation of the analyte of interest.
27. The method of claim 26, wherein the second time-correlated data comprises data from a ketone sensor.
28. The analyte monitoring system of claim 27, wherein the second time-correlated data comprises data indicating β-hydroxybutyrate.
29. The method of claim 28, wherein making the determination further comprises periodically recalculating ketone-on-board based on the data indicating β-hydroxybutyrate.
30. The method of claim 28, wherein making the determination further comprises selecting the message for output indicating that a patient wearing the sensor control device should conduct a ketone test, based on the data from the sensor for β-hydroxybutyrate exceeding a predetermined level.
31. The method of claim 25, wherein the second time-correlated data comprises data from a sensor for β-hydroxybutyrate, wherein an input device is operatively coupled to at least one of the reader device or the sensor control device is configured for receiving a ketone test result, wherein making the determination further comprises correcting an estimate of β-hydroxybutyrate based on the ketone test result.
32. The method of claim 25, wherein an input device is operatively coupled to at least one of the reader device or the sensor control device configured for receiving information defining insulin dosing by a patient wearing the sensor control device, and making the determination further comprises correcting an estimate of the patient's plasma insulin state.
33. The method of claim 25, wherein an input device is operatively coupled to at least one of the reader device or the sensor control device configured for receiving a ketone test result, further comprising automatically executing an insulin dose calculation algorithm in response to receiving the ketone test result.
34. The method of claim 25, wherein the indication of the determination is an alert condition for one analyte, and wherein the alert condition is based on more than one analyte.
35. The method of claim 34, wherein the distinguishing further comprises determining whether the first time-correlated data indicates a condition characterized by glucose variability below a threshold, high glucose for less than a maximum time threshold, and a ketone level greater than a predetermined threshold.
36. The method of claim 35, wherein the determining comprises at least one of suppressing a high ketone alert or reducing an urgency of a high ketone alert.
37. The method of claim 35, wherein the determining comprises causing the message to indicate a time period achieving high ketone levels.
38. The method of claim 34, wherein the distinguishing comprises determining whether the first time-correlated data indicates a condition characterized by glucose variability above a threshold, high glucose for greater than a maximum time threshold, and a ketone level greater than a predetermined threshold, and if the condition is detected, performing increasing at least one of a frequency or urgency of a message indicating potential occurrence of euglycemic DKA in the patient wearing the sensor control device.
39. The method of claim 25, wherein making the determination further comprises estimating whether a patient wearing the sensor control device is a person with Type 1 diabetes mellitus taking an SGLT-2 inhibitor based on comparing a ketone estimate based on the first time-correlated data with a ketone level indicated by the second time-correlated data.
40. The method of claim 39, wherein making the determination comprises determining whether the ketone estimate is consistently lower than the indicated ketone level, and if so, determining the message comprising an indication that the patient should consult with their health care provider regarding use of the SGLT-2 inhibitor.
41. The method of claim 40, wherein making the determination further causing the message to comprise an indication that the lack of a high glucose alert may not achieve the intended purpose.
42. The method of claim 25, further comprising wirelessly transmitting the first and the second data from the sensor control device to the reader device.

## Claims

1. An analyte monitoring system, comprising:
a sensor control device including an analyte sensor, wherein the analyte sensor includes at least a portion configured to be inserted into a user's body, wherein the sensor control device is configured to collect first time-correlated data indicative of a glucose level and second time-correlated data indicative of a ketone level, and wherein the sensor control device is operatively coupled to a first processing circuitry, and a first non-transitory memory;
a medication delivery device comprising an insulin pump; and
a reader device comprising second processing circuitry and a second non-transitory memory,
wherein at least one of the first or the second non-transitory memory includes instructions that, when executed, cause at least one of the first or the second processing circuitry to perform:
making a determination based on the first and second time-correlated data of a correction to an analyte state estimate; and
causing automatic execution of an insulin dose calculation algorithm.

2. The analyte monitoring system of claim 1, wherein the instructions for making the determination further cause using a closed-loop state observer to reconcile measurements at each time step against the predicted values of an estimated state of an analyte of interest, to obtain a corrected state estimation of the analyte of interest.

3. The analyte monitoring system of claim 2, wherein the closed-loop state observer is a Kalman filter.

4. The analyte monitoring system of any one of claims 1 to 3, wherein making the determination comprises using a dynamic model having model states corresponding to glucose, effective insulin, and ketones.

5. The analyte monitoring system of claim 4, wherein the dynamic model at any continuous time instance is described as functions of the current analyte state values and known or estimated external inputs.

6. The analyte monitoring system of any one of claims 1 to 5, wherein the second time-correlated data comprises data from a ketone sensor.

7. The analyte monitoring system of claim 6, wherein the second time-correlated data comprises data indicating β-hydroxybutyrate and, optionally, the instructions for making the determination further cause periodically recalculating ketone-on-board based on the data indicating β-hydroxybutyrate.

8. The analyte monitoring system of any preceding claim, wherein the reader device or the sensor control device is configured for receiving or calculating information defining insulin dosing for a patient wearing the sensor control device, wherein the instructions for making the determination cause correcting an estimate of the patient's plasma insulin state.

9. The analyte monitoring system of claim 8, wherein the correction of the estimate of the patient's plasma insulin state is performed using a closed-loop state observer, optionally a Kalman filter.

10. The analyte monitoring system of any preceding claim, wherein the insulin dosing is in the form of a delivery rate and/or a bolus dose from the insulin pump.

11. The analyte monitoring system of any preceding claim, wherein the instructions cause determining whether the first time-correlated data indicates a condition comprising glucose variability below a threshold, high glucose for less than a maximum time threshold, and a ketone level greater than a predetermined threshold.

12. The analyte monitoring system of any preceding claim, wherein the analyte sensor is a glucose sensor, wherein the sensor control device further includes a ketone sensor, and the glucose and ketone sensors are configured to sense analyte levels at the same localized site of insertion.

13. The analyte monitoring system of any preceding claim, wherein the instructions cause:
calculation of insulin delivery amounts and durations based on the analyte level measurements obtained from the sensor control device;
and delivery of the insulin to the patient.

14. A computer-implemented method comprising:
collecting, by a sensor control device, first time-correlated data indicative of a glucose level and second time-correlated data indicative of a ketone level, wherein the sensor control device includes an analyte sensor at least a portion of which is inserted into a user's body;
making a determination based on the first and second time-correlated data of a correction to an analyte state estimate; and
causing automatic execution of an insulin dose calculation.

15. A computer program, computer program product, or computer readable medium comprising instructions which, when executed by a processor, cause the processor to perform the method of claim 14.
